# EUROPEAN PATENT SPECIFICATION

(11) **EP 3 684 343 B1**
(45) Date of publication and mention of the grant of the patent: **13.09.2023**
(21) Application number: 18858164.9
(22) Date of filing: 20.09.2018
(51) Int. Cl.: A61K 38/18, A61K 38/20, A61K 9/51, A61K 9/16, A61K 31/573, A61P 27/16

(54) **IMPROVED SUPRAPARTICLES**
VERBESSERTE SUPRAPARTIKEL
SUPERPARTICULES AMÉLIORÉES

(30) Priority: 20.09.2017 AU 2017903828; 20.09.2017 AU 2017903829; 11.07.2018 AU 2018902513
(43) Date of publication of application: 29.07.2020
(73) Proprietor: The Bionics Institute of Australia, East Melbourne, Victoria 3002 (AU); The University of Melbourne, Parkville, Victoria 3010 (AU)
(72) Inventor: WISE, Andrew, East Melbourne Victoria 3002 (AU); CARUSO, Frank, Parkville Victoria 3010 (AU); BJORNMALM, Mattias, Parkville Victoria 3010 (AU); MA, Yutian, East Melbourne ^Victoria 3002 (AU)
(74) Representative: Dehns
(86) International application number: PCT/AU2018/051029
(87) International publication number: WO 2019/056062

(56) References cited:
- WO-A1-2015/051364
- US-A1- 2015 051 364
- MAINA et al.: "Mold-Templated Inorganic-Organic Hybrid Supraparticles for Codelivery of Drugs", Biomacromolecules, vol. 15, no. 11, 2014, pages 4146-4151, XP055584226,
- WISE et al.: "Improved Auditory Nerve Survival with Nanoengineered Supraparticles for Neurotrophin Delivery into the Deafened Cochlea", PLoS One, vol. 11, no. 10, 2016, XP055584222,
- WANG et al.: "Mesoporous Silica Supraparticles for Sustained Inner-Ear Drug Delivery", Small, vol. 10, no. 21, 2014, pages 4244-4248, XP055584223,
- MA et al.: "Gel-Mediated Electrospray Assemby of Silica Supraparticles for Sustained Drug Delivery", ACS Applied Materials & Interfaces, vol. 10, no. 37, 7 September 2018 (2018-09-07), pages 31019-31031, XP055584228,
- WISE et al.: "Drug delivery to the inner ear", Journal of Neural Engineering, vol. 9, no. 6, 2012, XP020234320, DOI: doi:10.1088/1741-2560/9/6/065002

## Description

### FIELD OF THE INVENTION

The present disclosure relates to compositions comprising improved supraparticles loaded with high levels of payload and methods for their production. Such supraparticle compositions may be used in a range of therapeutic applications.

### BACKGROUND OF THE INVENTION

Mesoporous silicas are porous materials with extremely high surface areas which have been widely used to encapsulate various compounds and for the template synthesis of diverse nanostructured materials. While, porous materials of this type are of interest in a diverse range of applications such as drug delivery, various issues such as efficient loading and sustained drug delivery remain unresolved.

It would be desirable to provide new materials of this type as well as new methods of making such materials, as it would be expected that the new materials may have a number of interesting properties.

Accordingly, improved supraparticles are required.

Maina et al. "Mold-Templated Inorganic-Organic Hybrid Supraparticles for Codelivery of Drugs", Biomacromolecules, vol. 15, no. 11, 2014, pages 4146-4151 reports on a facile and robust mold-templated technique for assembly of mesoporous silica (MS) supraparticles, and demonstrates their potential as co-delivery vehicles for both brain-derived neurotrophic factor (BDNF) and dexamethasone (DEX).

Wise et al. "Improved Auditory Nerve Survival with Nanoengineered Supraparticles for Neurotrophin Delivery into the Deafened Cochlea", PLoS One, vol. 11, no. 10, 2016, article 0164867 reports on the safety and efficacy of a drug delivery system for the cochlea using nanoengineered silica supraparticles.

Wang et al. "Mesoporous Silica Supraparticles for Sustained Inner-Ear Drug Delivery", Small, vol. 10, no. 21, 2014, pages 4244-4248 reports on the preparation of mesoporous silica supraparticles (MS-SPs) via self-assembly of mesoporous silica nanoparticles under capillary force action in confined droplets

### SUMMARY OF THE INVENTION

The present invention is based on the unexpected production of supraparticles that can be loaded with high levels of payload. The present inventors have also identified that supraparticles of use in the present invention appear to have unique pore sizes which may account for the increased levels of loading. Accordingly, in a first aspect, the present invention provides a composition comprising a supraparticle, said supraparticle comprising mesoporous silica nanoparticles and at least one payload, wherein said payload is at least 1.5 µg of a neurotrophic factor.. In an example, the supraparticle of use in the invention comprises at least 5 µg of payload. In another example, the supraparticle of use in the invention comprises at least 8 µg of payload. In another example, the supraparticle of use in the invention comprises at least 10 µg of payload. In another example, the supraparticle of use in the invention comprises 1.5 µg to 10 µg of payload.

In an example, the supraparticle of use in the invention comprises pores having a diameter of at least 60 nm. In another example, the supraparticle of use in the invention comprises pores having a diameter of at least 100 nm. In another example, the supraparticle of use in the invention comprises pores having a diameter of at least 60 - 200 nm. In another example, the supraparticle of use in the invention comprises pores having a diameter of at least 50- 100 nm.

In another example, the supraparticles of use in the invention have a disordered pore structure.

In an example, the supraparticle of use in the invention is comprised of mesoporous silica nanoparticles having a bimodal pore structure. In an example, the nanoparticles' bimodal pore structure has a large pore diameter greater than 30 nm.

In another example, the payload of the supraparticle of use in the invention is a neurotrophin. In an example, the neurotrophic factor has an isoelectric point between 9 and 10. In another example, the payload is neurotrophin-3. In another example, the payload is BDNF or neurotrophin-3.

In another example, the supraparticle of use in the invention comprises at least 2, at least 3, at least 4, at least 5 different payloads, one payload being a neurotrophic factor. In another example, the supraparticle of use in the invention comprises at least two payloads, one payload being a neurotrophin.

In other examples, a composition disclosed herein comprises at least two, at least three, at least four, at least five, at least six, at least seven, at least eight, at least nine, at least 10 supraparticles. In these examples, the supraparticles may comprise different payloads.

In an example, supraparticles of use in the invention may be produced by electrospraying a composition comprising mesoporous silica nanoparticles into a di-cationic aqueous solution. In an example, the composition comprising mesoporous silica nanoparticles also comprises an Alginic acid or a polysaccharide derivative thereof. Accordingly, in an example, supraparticles of use in the invention may be produced by electrospraying a composition comprising mesoporous silica nanoparticles and Alginic acid or a polysaccharide derivative thereof into a di-cationic aqueous solution. In accordance with the invention, supraparticles produced in these examples may be loaded with 1.5 µg to 15 µg of a payload such as a neurotrophin. In these examples, supraparticles of use in the invention may also be subject to calcination to remove alginic acid before being loaded with payload. In an example, calcination is performed at around 650 °C. In an example, the calcination is performed for about 6 to about 30 hours. In an example, the Alginic acid is Alginic acid sodium salt. Accordingly, in an example, the present invention encompasses compositions comprising supraparticles as defined herein, wherein the supraparticles are manufactured by electrospraying a composition comprising mesoporous silica nanoparticles and alginic acid or a polysaccharide derivative thereof into a di-cationic aqueous solution. In this example, the supraparticles of use in the invention may be subjected to calcination to remove alginic acid. In an example, the calcination is performed at around 650°C. In an example, the calcination is performed for about 6 to about 30 hours.

The present inventors have also identified that supraparticles disclosed herein can release payload over a relatively long period of time. Furthermore, supraparticles as defined herein can be provided in various formulations to manipulate release of payload. For example, the present inventors have identified that supraparticles as defined herein can release their respective payload over a prolonged duration. They have also identified that supraparticles as defined herein can be provided in various formulations to accelerate or slow the release of payload. Accordingly, in an example, a supraparticle of use in the invention or composition of the invention may be provided as a slow release formulation. In an example, the slow release formulation is a foam or a gel. In an example, a composition of the invention comprises a glycogen hydrogel. In another example, a composition of the invention comprises Alginic acid [(C₆H₈O₆)ₙ] or a polysaccharide derivative thereof. In another example, a composition of the invention comprises Alginic acid sodium salt [Na(C₆H₈O₆)ₙ] or a polysaccharide derivative thereof..

In an example, a composition of the invention is provided as an accelerated release formulation. In an example, the accelerated release formulation is a foam or a gel. In an example, the gel or foam is thermoreversible. For example, the accelerated formulation may comprise a supraparticle as defined herein and a PF127 based hydrogel.

The present invention further provides a composition as defined herein for use in treating a disease or disorder. In an example, the disease or disorder is hearing loss. In an example, the hearing loss is characterised as sensorineural hearing loss (SNHL), presbycusis or noise induced.

The present invention further provides a composition as defined herein for use in promoting survival of spiral ganglion neurons in an ear of a subject. Said use may comprise administering the composition defined herein to a subject. For example, the composition can be administered to a subject's ear.

The present invention further provides a method of manufacturing a composition as defined herein comprising electrospraying a composition comprising mesoporous silica nanoparticles and alginic acid into a di-cationic aqueous solution and loading the supraparticles so-produced with at least 1.5 µg of a neurotrophic factor. In an example, such a method further comprise subjecting the supraparticles to calcination to remove alginic acid. In an example, calcination is performed at around 650 °C. In an example, the calcination is performed for about 6 to about 30 hours.

Any example herein shall be taken to apply *mutatis mutandis* to any other example unless specifically stated otherwise.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

The disclosure is hereinafter described by way of the following non-limiting Examples and with reference to the accompanying drawings.

### BRIEF DESCRIPTION OF THE ACCOMPANYING DRAWINGS

**Figure 1****.** Procedure of making MS-SPs.
**Figure 2****.** Zeta potential of MS-SPs as a function of different pH values. Zeta potential measurements were carried out in 10mM sodium acetate buffer (pH 4), 10mM phosphate buffer (pH 6), 10mM HEPES buffer (pH 8) and 10mM sodium bicarbonate buffer (pH 10).
**Figure 3****.** Confocal microscopy images of MS-SPs loaded with FITC-lysozyme (green). a, b) MS-SPs at different magnifications. c) Inside of a fragmented MS-SP loaded with FITC-lysozyme.

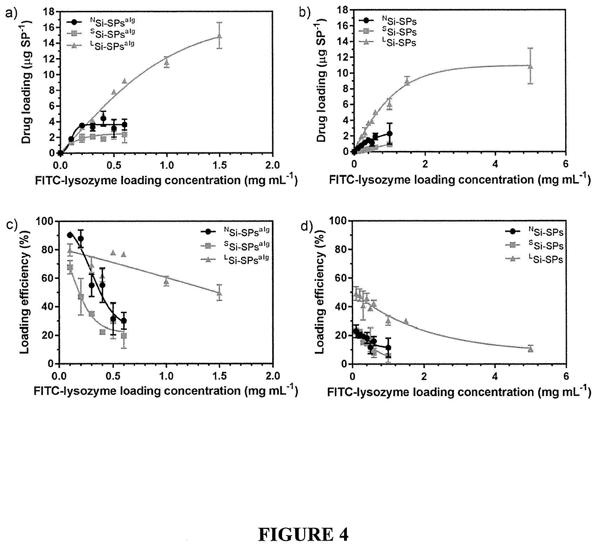

**Figure 4****.** Drug loading after 3 days (72 h) incubation time using different FITC-lysozyme loading concentration. a) The adsorbed amount of FITC-lysozyme into non-porous MS-SPs^{alg} (black circles), small pore MS-SPs^{alg} (blue squares) and MS-SPs^{alg} (red triangles) versus the FITC-lysozyme loading concentration. b) The adsorbed amount of FITC-lysozyme into non-porous MS-SPs (black circles), small pore MS-SPs (blue squares) and MS-SPs (red triangles) versus the FITC-lysozyme loading concentration. c) The FITC-lysozyme loading efficiency in MS-SPs^{alg} and d) MS-SPs. Data presented is average of three replicates, each using five SPs, and error bars represent standard deviation.
**Figure 5****.** FITC-lysozyme loading and release behaviour from MS-SPs with different diameters. a) FITC-lysozyme loading amount, b) *In-vitro* FITC-lysozyme release profile, c) the value of FITC-lysozyme released at each individual time point, d) *In-vitro* FITC-lysozyme release standard curve from MS-SPs with the diameter of 200, 550, 650, 1000 µm.
**Figure 6****.** Cell viability of human brain glioblastoma cells (U87MG cell line) incubated with different numbers of MS-SPs for 48 h. Ethanol added to cells with inserts was prepared as negative (cytotoxic) control and untreated cells represent 100% viability. Data presented is average of four samples, and error bars represent standard deviation.

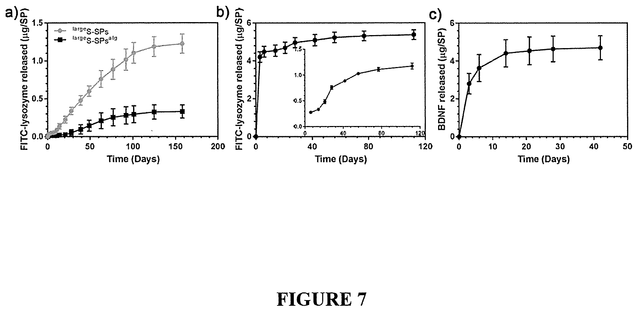

**Figure 7****.** *In-vitro* cumulative drug release profile, a). *In-vitro* FITC-lysozyme (0.2 mg mL⁻¹, drug loading for 3 days) release from MS-SPs (red circle), and MS-SPs^{alg} (black triangle). Data presented is average of three replicates, each using ten SPs, and error bars represented standard deviation. b) *In-vitro* FITC-lysozyme (1.0 mg mL⁻¹, drug loading for 3 days) release from MS-SPs. The inset in (b) is the *in-vitro* FITC-lysozyme release profile starting from 6 days. c) *In-vitro* BDNF release from MS-SPs (1.0 mg mL⁻¹, drug loading for 3 days). BDNF ELISA (Abcam) was used for the measurement of BDNF concentration.
**Figure 8****.** The value of FITC-lysozyme released in each individual time point for MS-SPs^{alg} and MS-SPs (loading conditions: 100 µL of 0.2 mg mL⁻¹ of FITC-lysozyme loaded, 3 days loading time). Data presented is average of three replicates, each using ten supraparticles, and error bars represent standard deviation.
**Figure 9****.** The value of FITC-lysozyme released in each individual time point for MS-SPs (loading conditions: 100 µL of 1.0 mg mL⁻¹ of FITC-lysozyme loaded over 3 days). Drug loading was 6.49 ± 0.48 µg per supraparticle. Data presented is average of three replicates, each using ten MS-SPs, and error bars represent standard deviation.
**Figure 10****.** The absolute value in each individual time points for in-vitro BDNF drug studies (loading conditions: 1.0 mg mL⁻¹ BDNF loaded over 3 days). Data presented is average of three replicates, each using one MS-SP, and error bars represent standard deviation.
**Figure 11.** a) MicroBCA assay for the measurement of BDNF from MS-SPs. b) The value of BDNF released at each individual time point of using MS-SPs. Drug loading is 6.82 µg per supraparticle. Data presented is average of three replicates, and error bars represent standard deviation.
**Figure 12.** a) *In-vitro* FITC-lysozyme release profile of MS-SPs (red circle), and MS-SPs^{alg} (black square) in PF127 hydrogel system. b) The value of FITC-lysozyme released at each individual time point for figure (a) (100 µL of 0.2 mg mL⁻¹ FITC-lysozyme loaded). Drug loading is 1.89 ± 0.08 µg per supraparticle and 1.93 ± 0.03 µg per supraparticle in MS-SPs and MS-SPs^{alg} respectively. Data presented is average of three replicates, each using ten supraparticles, and error bars represent standard deviation.
**Figure 13****.** *In vitro* release profile when 50% loaded (3.65 µg/SP)
**Figure 14****.** Degradation of MS-SPs after 150 days of *in-vitro* drug release studies. a) SEM image of MS-SPs after 150 days of incubation in PBS (pH 7.4) at 37 °C. b, c) SEM images of the surface structure of MS-SPs after 150 days of incubation in PBS (pH 7.4) at 37°C.
**Figure 15****.** Amount of neurotrophin-3 (% of loaded) and the total in µg is shown. After 1 week of implantation each SP contained ~2ug of neurotrophin-3 (~40%) of initial loaded amount.

### DETAILED DESCRIPTION OF THE INVENTION

### General Techniques and Selected Definitions

Unless specifically defined otherwise, all technical and scientific terms used herein shall be taken to have the same meaning as commonly understood by one of ordinary skill in the art (e.g., physiology, clinical studies, molecular biology, high surface area molecules, electrospraying and biochemistry).

Unless otherwise indicated, techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

As used in this specification and the appended claims, terms in the singular and the singular forms "a," "an" and "the," for example, optionally include plural referents unless the content clearly dictates otherwise. Thus, for example, reference to "a supraparticle" optionally includes a plurality of supraparticles.

As used herein, the term "about", unless stated to the contrary, refers to +/- 10%, more preferably +/- 5%, more preferably +/- 1%, of the designated value.

The term "and/or", e.g., "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As used herein, the term "treatment" refers to clinical intervention designed to alter the natural course of the individual or cell being treated during the course of clinical pathology. Exemplary desirable effects of treatment include reduction in symptoms associated with a disorder being treated. In the context of treating hearing loss, desirable effects of treatment include decreasing the rate of hearing loss and ameliorating or palliating hearing loss. An individual is successfully "treated", for example, if one or more symptoms associated with the disorder are mitigated or eliminated.

A "therapeutically effective amount" refers to at least the minimum amount required to effect a measurable improvement of a particular disorder. A therapeutically effective amount can also include at least the minimum amount required to effect a measurable improvement in a subjects disorder. A therapeutically effective amount can be provided in one or more administrations. The therapeutically effective amount may vary according to the severity of the disorder being treated and also according to the weight, age, racial background, sex, health and/or physical condition of a subject being treated. Typically, the effective amount will fall within a relatively broad range (e.g. a "dosage" range) that can be determined through routine trial and experimentation by a medical practitioner. The therapeutically effective amount can be administered in a single dose or in a dose repeated once or several times over a treatment period.

### Payload

Supraparticles of use in the invention, can comprise various payloads as long as the payload of the supraparticle comprises at least 1.5 µg of a neurotrophic factor. A "payload" can be any agent useful for treating a disorder. Examples of agents include biological products such as polynucleotides, antibodies, monoclonal antibodies, antibody fragments, antibody drug conjugates, proteins, biologically active proteins, fusion proteins, recombinant proteins, peptides, polypeptides, synthesized polypeptides, vaccines, therapeutic serums, viruses, polynucleotides, cells such as stem cells or parts thereof as well as small molecules.

Exemplary viral payloads can comprise appropriately modified retrovirus, Adenovirus (AdV), Adeno-associated virus (AAV) or a recombinant form such as recombinant adeno-associated virus (rAAV) and derivatives thereof such as self-complementary AAV (scAAV) and non-integrating AV. Other exemplary viral therapeutic payloads can comprise herpes simplex virus (HSV), lentivirus, vaccina and vesicular stomatitis virus (VSV). For example, the viral therapeutic payload can comprise AAV. Various AAV serotypes are known and may be suitable viral payloads. In an example, the AAV is serotype 2. In another example, the AAV is serotype 1. In other examples, the AAV is serotype 3, 4, 7, 8, 9, 10, 11, 12 or 13.

In an example, a small molecule payload is a neurotransmitter. The term "neurotransmitter" is used in the context of the present disclosure to refer to a substance that transmits signal(s) across a chemical synapse from one cell to another. Generally, a neurotransmitter transmits signal(s) across a chemical synapse from one neuron to a target cell such as another neuron, muscle cell or gland cell. In another example, the small molecule payload is a receptor agonist. The term "agonist" is used in the context of the present disclosure to refer to a substance which initiates a physiological response when combined with a receptor. In another example, the small molecule payload is a receptor antagonist. The term "antagonist" is used in the context of the present disclosure to refer to a substance which interferes with or inhibits the physiological action of a receptor.

Exemplary polynucleotide payloads include antisense polynucleotides, double stranded DNA (dsDNA) or double stranded RNA (dsRNA). In one example, the dsDNA or dsRNA is an aptamer. In another example, the dsRNA is a siRNA, miRNA or shRNA.

Exemplary antibody payloads and fragments thereof include human antibodies, humanized antibodies, chimeric antibodies, single chain antibodies, diabodies, triabodies, tetrabodies or single domain antibodies. In an example, the antibody can be bi-specific, an antibody-drug conjugate or a biosimilar antibody. Other exemplary polypeptides include cytokines, chemokines, hormones and blood coagulation factors. In an example the polypeptide payload is an enzyme. Exemplary enzymes include proteases, lipases, asparaginases, liprotamases, tissue plasminogen activators, collagenases, glutaminases, hyaluronidases, streptokinases, uricases, urokinases or nucleases, such as a programmable nuclease. In an example, the enzyme can be a DNA methyltransferase. In an example, the enzyme can be a programmable nuclease targeted to introduce a genetic modification into a gene or a regulator region thereof. For example, the programmable nuclease can be a RNA-guided engineered nuclease (RGEN). In an example, the RGEN is from an archaeal genome or may be a recombinant version thereof. In another example, the RGEN may be from a bacterial genome or is a recombinant version thereof. In another example, the RGEN is from a Type I (CRISPR)-cas (CRISPR-associated) system. In another example, the RGEN is from a Type II (CRISPR)-cas (CRISPR-associated) system. In another example, the RGEN is from a Type III (CRISPR)-cas (CRISPR-associated) system. In an example, the nuclease is from a class I RGEN or a class II RGEN.

In another example, the therapeutic payload is a DNA methylation inhibitor, a histone acetyl transferase inhibitor or a histone deacetylase inhibitor.

In another example, the therapeutic payload may be an antigen which stimulates an immune response in a subject. Exemplary antigens include proteins, peptides, polysaccharides or oligosaccharides (free or conjugated to a protein carrier) or mixtures thereof. Other exemplary antigens include cells or parts thereof or a viral particle or a part thereof.

In an example, the therapeutic payload is an antineoplastic agent.

Other exemplary therapeutic payloads include agonists or antagonists to membrane receptors in the inner ear. Such therapeutic payloads may modulate neurotransmission. In another example, the therapeutic payload is a neurological agent useful for treating one or more neurological disorders, such as Alzheimer's disease, Parkinson's disease, Epilepsy or multiple sclerosis.

The term neurotrophic factor is used in the context of the present invention to refer to molecules that enhance the growth or survival potential of any cells including those from the auditory system. For example, neurotrophic factors of use in the invention can enhance growth or survival of cells from the auditory system or their synaptic connections located in the inner ear, middle ear or vestibular system. Exemplary cells include spiral ganglion neurons (SGNs), hair cells, including inner and outer ear hair cells, cochlear glial cells and Schwann cells. Exemplary synaptic connections include connections between hair cells, hair cells and SGNs or other neurons discussed above. Other examples include neuronal cell bodies in Rosenthal's canal or their synaptic connections, neurons or synaptic connections in the upper middle cochlear regions and/or nerve fibres in the osseous spiral lamina.

Exemplary neurotrophic factors can include agents discussed above with known therapeutic efficacy for directly or indirectly enhancing survival of cells from the auditory system and/or their synaptic connections.

In an example, the neurotrophic factor of use in the invention is a neurotrophic peptide. Exemplary neurotrophic peptides include, brain derived neuroptrophic factor (BDNF), nerve growth factor, neurotrophin-3, neurotrophin-4, members of the ciliary neurotrophic factor (CNTF) family such as CNTF, Leukemia inhibitory factor (LIF), Interleukin-6 (IL-6), Glia maturation factor (GMF), insulin growth factor-1 (IGF-1), Neuregulin 1, Neuregulin 2, Neuregulin 3 and Neuregulin 4, vascular endothelial growth factor (VEGF), members of the Glial Cell Derived Neurotrophic Factor (GDNF) family such as GDNF, neurturin (NRTN), artemin (ARTN), and persephin (PSPN), ephrins such as A1, A2, A3, A4, A5, B1, B2 and B3, insulin growth factor-1 (IGF-1) and interleukins such as IL-11.

In an example, the neurotrophic factor of use in the invention is selected from the group consisting of brain derived neurotrophic factor (BDNF), nerve growth factor, neurotrophin-3, neurotrophin-4, ciliary neurotrophic factor (CNTF), Glial Cell Derived Neurotrophic Factor (GDNF) and IL-11..

One cause of spiral ganglion neuron degeneration is the loss of the endogenous supply of neurotrophins. Thus, in an example, the neurotrophic factor of use in the invention is a neurotrophin. The term "neurotrophin" is used in the context of the present disclosure to refer to proteins that induce the survival, development and/or function of neurons and/or their synaptic connections. Exemplary neurotrophins are discussed above and include BDNF, nerve growth factor, neurotrophin-3 and neurotrophin-4. Accordingly, in an example, the supraparticle comprises BDNF. In another example, the supraparticle comprises nerve growth factor. In another example, the supraparticle comprises neurotrophin-3. In another example, the supraparticle comprises neurotrophin-4. In an example, supraparticles can comprise at least two different neurotrophins. In other examples, supraparticles can comprise at least three or four different neurotrophins.

Therapeutic efficacy may be improved by administering supraparticles comprising multiple different therapeutic payloads. Thus, in an example, supraparticles of use in the invention can comprise at least two, at least three, at least four, at least five, at least six, at least seven, at least eight, at least nine, at least 10 different therapeutic payloads, as long as the payload of the supraparticle comprises at least 1.5 µg of a neurotrophic factor.

For example, supraparticles of use in the invention can comprise at least two different neurotrophic factors. In other examples, supraparticles of use in the invention can comprise at least three, at least four, at least five different neurotrophic factors. In these examples, various combinations of neurotrophic factors such as neurotrophins are contemplated. Exemplary combinations of neurotrophic factors include BDNF and nerve growth factor, BDNF and neurotrophin-3, BDNF and neurotrophin-4, BDNF and CNTF, BDNF and GDNF, BDNF and IL-11, neurotrophin-3 and neurotrophin-4, neurotrophin-3 and CNTF, neurotrophin-3 and CNTF, neurotrophin-3 and GDNF, neurotrophin-3 and IL-11, neurotrophin-4 and CNTF, neurotrophin-4 and GDNF, neurotrophin-4 and IL-11, CNTF and GDNF, CNTF and IL-11, GDNF and IL-11, BDNF, neurotrophin-3 and neurotrophin-4, BDNF, neurotrophin-3 and CNTF, BDNF, neurotrophin-3 and GDNF, BDNF, CNTF and GDNF, BDNF, CNTF and IL-11, neurotrophin-3, neurotrophin-4 and CNTF, neurotrophin-3, neurotrophin-4 and GDNF, neurotrophin-3, CDNF and GDNF, neurotrophin-3, neurotrophin-4 and IL-11, neurotrophin-4, CNTF and GDNF, neurotrophin-4, CNTF and IL-11, BDNF, neurotrophin-3, neurotrophin-4 and CNTF, BDNF, neurotrophin-3, neurotrophin-4 and GDNF, BDNF, neurotrophin-3, CNTF and GDNF, BDNF, neurotrophin-4, CNTF and GDNF, BDNF, BDNF, neurotrophin-3, neurotrophin-4 and IL-11, neurotrophin-3, neurotrophin-4, CNTF and GDNF, neurotrophin-3, neurotrophin-4, CNTF and IL-11.

Other exemplary neurotrophic factors include antibodies or other binding proteins such as anti-Tropomyosin receptor kinase (TrK) B, anti-TrK C or binding proteins that interact with p75 neurotrophin receptor. For example, p75 neurotrophin receptor antagonists. In another example, neurotrophic factors include nucleic acids. For example, the neurotrophic factor can comprise a gene therapy, silencing RNA such as a siRNA or miRNA, expression constructs such as DNA plasmids comprising a nucleic acid of interest. In an example, the neurotrophic factor is an expression construct comprising a nucleic acid encoding an opsin(s).

In another example, a supraparticle of use in the invention can comprise an expression vector comprising an opsin and any one or more of BDNF, nerve growth factor, neurotrophin-3, neurotrophin-4 and GDNF. In another example, a supraparticle of use in the invention can can comprise an antibody such as anti-Tropomyosin receptor kinase (TrK) B or anti-TrK C and any one or more of BDNF, nerve growth factor, neurotrophin-3, neurotrophin-4, GDNF and IL-11.

As long as the payload of the supraparticle comprises at least 1.5 µg of a neurotrophic factor, supraparticles of use in the invention can comprise at least two, at least three, at least four, or at least five different therapeutic payloads wherein at least one therapeutic payload is a neurotrophic factor. For example, such supraparticles can comprise at least three different therapeutic payloads wherein two therapeutic payloads are neurotrophic factors. In another example, such supraparticles can comprise at least four different therapeutic payloads wherein three therapeutic payloads are neurotrophic factors. In these examples, the further therapeutic payload need not enhance the growth or survival potential of cells from the auditory system but rather can provide another therapeutic benefit. For example, the further therapeutic payload may suppress a subject's immune system following administration of supraparticles. In another example, supraparticles of use in the invention may comprise a payload that reduces survival of cells from the auditory system or their synaptic connections and a neurotrophic factor(s). In this example, the neurotrophic factor may alleviate the reduction in survival of cells from the auditory system or their synaptic connections caused by the further therapeutic payload. In an example, supraparticles of use in the invention may comprise antineoplastic agents including cisplatinum or related compounds, antibiotics including aminoglycosides such as tobramycin or related compounds, loop diuretics such as furosemide, antimetabolites such as methotrexate, salicylates such as aspirin or a radioactive moiety and a neurotrophic factor(s). For example, a supraparticle can comprise an antibiotic and any one or more of BDNF, nerve growth factor, neurotrophin-3, neurotrophin-4, GDNF and IL-11.

Depending on the site of administration the payload may need to diffuse from the middle ear to the inner ear or vestibular system. This may occur via diffusion of the payload across the round or oval widows. Thus, in an example, supraparticles of use in the invention may comprise a molecule(s) that assist diffusion of the payload across the round and oval windows to the inner ear and/or vestibular system.

In an example, the payload of the supraparticles of use in the invention has an isoelectric point greater than 7. In another example, the payload has an isoelectric point greater than 8. In another example, the payload has an isoelectric point greater than 9. In another example, the payload has an isoelectric point greater than 10. In another example, the payload has an isoelectric point between 7 and 10. In another example, the payload has an isoelectric point between 7 and 9. In another example, the payload has an isoelectric point between 8 and 10. In another example, the payload has an isoelectric point between 9 and 10.

Supraparticles comprising a payload may be referred to as loaded supraparticles in the context of the present disclosure. Methods of producing loaded supraparticles are not particularly limited so long as the resulting supraparticle can be loaded with at least 1.5 µg of a neurotrophic factor payload. Preferably, the resulting supraparticle can deliver the payload to an ear of a subject. Exemplary methods of loading are reviewed in Wang et al. (2009) J. Mater. Chem. 19, 6451 and include payload encapsulation and entrapment. In one non-limiting example, supraparticles of use in the invention may be loaded by contacting the supraparticle with an aqueous solution of the payload followed by a period of incubation. The payload solution can contain an excess of the amount of payload to be loaded onto the supraparticle and incubation can occur at room temperature. Agitation of the solution containing the supraparticle and the payload may be used to enhance loading of the payload.

One of skill in the art will appreciate that the required level of payload will likely be influenced by the payload itself and the indication being treated according to the present disclosure.

### Supraparticles

The term "supraparticle" ("SP") is used in the context of the present disclosure to refer to agglomerated particles comprising a network of pores. The network of pores provides supraparticles with a large pore volume and surface area for carrying a payload. A large pore volume and surface area is advantageous as it can enhance the amount of payload that can be carried by supraparticles. In an example, supraparticles according to the present disclosure are agglomerated nanoparticles.

Supraparticles of use in the invention comprise mesoporous silica nanoparticles.

Supraparticles of use in the present invention comprise at least 1.5 µg of payload. In another example, supraparticles comprise at least 2.0 µg of payload.

In another example, supraparticles comprise at least 2.5 µg of payload. In another example, supraparticles comprise at least 2.6 µg of payload. In another example, supraparticles comprise at least 2.7 µg of payload. In another example, supraparticles comprise at least 2.7 µg of payload. In another example, supraparticles comprise at least 2.8 µg of payload. In another example, supraparticles comprise at least 2.9 µg of payload. In another example, supraparticles comprise at least 3.0 µg of payload. In another example, supraparticles comprise at least 3.1 µg of payload. In another example, supraparticles comprise at least 3.2 µg of payload. In another example, supraparticles comprise at least 3.3 µg of payload. In another example, supraparticles comprise at least 3.4 µg of payload. In another example, supraparticles comprise at least 3.5 µg of payload. In another example, supraparticles comprise at least 3.6 µg of payload. In another example, supraparticles comprise at least 3.7 µg of payload. In another example, supraparticles comprise at least 3.8 µg of payload. In another example, supraparticles comprise at least 3.9 µg of payload. In another example, supraparticles comprise at least 4.0 µg of payload. In another example, supraparticles comprise at least 4.5 µg of payload. In another example, supraparticles comprise at least 5.0 µg of payload. In another example, supraparticles comprise at least 5.5 µg of payload. In another example, supraparticles comprise at least 6.0 µg of payload. In another example, supraparticles comprise at least 6.5 µg of payload. In another example, supraparticles comprise at least 7.0 µg of payload. In another example, supraparticles comprise at least 7.5 µg of payload. In another example, supraparticles comprise at least 8.0 µg of payload. In another example, supraparticles comprise at least 8.5 µg of payload. In another example, supraparticles comprise at least 9.0 µg of payload. In another example, supraparticles comprise at least 9.5 µg of payload. In another example, supraparticles comprise at least 10 µg of payload. In another example, supraparticles comprise at least 10.5 µg of payload. In another example, supraparticles comprise at least 11 µg of payload. In another example, supraparticles comprise at least 11.5 µg of payload. In another example, supraparticles comprise at least 12 µg of payload. In another example, supraparticles comprise at least 15 µg of payload. In another example, supraparticles comprise at least 20 µg of payload.

For example, supraparticles of use in the invention can comprise at least 6 µg of payload.

In another example, supraparticles of use in the invention can comprise between about 2.5 and 10 µg of payload. In another example, supraparticles can comprise between about 3 and 10 µg of payload. In another example, supraparticles can comprise between about 4 and 10 µg of payload. In another example, supraparticles can comprise between about 5 and 10 µg of payload. In another example, supraparticles can comprise between about 6 and 10 µg of payload. In another example, supraparticles can comprise between about 5 and 15 µg of payload. In another example, supraparticles can comprise between about 5 and 20 µg of payload. In another example, supraparticles can comprise between about 8 and 20 µg of payload. In another example, supraparticles can comprise between about 8 and 15 µg of payload.

For example, supraparticles of use in the invention can comprise between about 6 and 8 µg of payload.

Supraparticles of use in the present invention comprise at least 1.5 µg of neurotrophic factor. In another example, supraparticles comprise at least 2.0 µg of neurotrophic factor. In another example, supraparticles comprise at least 2.5 µg of neurotrophic factor. In another example, supraparticles comprise at least 3.0 µg of neurotrophic factor. In another example, supraparticles comprise at least 3.5 µg of neurotrophic factor. In another example, supraparticles comprise at least 4.0 µg of neurotrophic factor. In another example, supraparticles comprise at least 5.0 µg of neurotrophic factor. In another example, supraparticles comprise at least 6.0 µg of neurotrophic factor. In another example, supraparticles comprise at least 7.0 µg of neurotrophic factor. In another example, supraparticles comprise at least 8.0 µg of neurotrophic factor. In another example, supraparticles comprise at least 9.0 µg of neurotrophic factor. In another example, supraparticles comprise at least 10 µg of neurotrophic factor. In another example, supraparticles comprise at least 10.5 µg of neurotrophic factor. In another example, supraparticles comprise at least 11 µg of neurotrophic factor. In another example, supraparticles comprise at least 11.5 µg of neurotrophic factor. In another example, supraparticles comprise at least 12 µg of neurotrophic factor. In another example, supraparticles comprise at least 15 µg of neurotrophic factor. In another example, supraparticles comprise at least 20 µg of neurotrophic factor.

For example, supraparticles of use in the invention can comprise at least 6 µg of neurotrophic factor.

In another example, supraparticles of use in the invention can comprise between about 2.5 and 10 µg of neurotrophic factor. In another example, supraparticles can comprise between about 5 and 10 µg of neurotrophic factor. In another example, supraparticles can comprise between about 6 and 10 µg of neurotrophic factor. For example, supraparticles can comprise between about 6 and 8 µg of neurotrophic factor. In another example, supraparticles can comprise between about 5 and 15 µg of neurotrophic factor. In another example, supraparticles can comprise between about 5 and 20 µg of neurotrophic factor. In another example, supraparticles can comprise between about 8 and 20 µg of neurotrophic factor. In another example, supraparticles can comprise between about 8 and 15 µg of neurotrophic factor.

In an example, supraparticles of use in the invention comprise at least 1.5 µg of neurotrophin. In another example, supraparticles comprise at least 2.0 µg of neurotrophin.

In another example, supraparticles of use in the invention comprise at least 2.5 µg of neurotrophin. In another example, supraparticles comprise at least 3.0 µg of neurotrophin. In another example, supraparticles comprise at least 3.5 µg of neurotrophin. In another example, supraparticles comprise at least 4.0 µg of neurotrophin. In another example, supraparticles comprise at least 5.0 µg of neurotrophin. In another example, supraparticles comprise at least 6.0 µg of neurotrophin. In another example, supraparticles comprise at least 7.0 µg of neurotrophin. In another example, supraparticles comprise at least 8.0 µg of neurotrophin. In another example, supraparticles comprise at least 9.0 µg of neurotrophin. In another example, supraparticles comprise at least 10 µg of neurotrophin. In another example, supraparticles comprise at least 10.5 µg of neurotrophin. In another example, supraparticles comprise at least 11 µg of neurotrophin. In another example, supraparticles comprise at least 11.5 µg of neurotrophin. In another example, supraparticles comprise at least 12 µg of neurotrophin. In another example, supraparticles comprise at least 15 µg of neurotrophin. In another example, supraparticles comprise at least 20 µg of neurotrophin.

For example, supraparticles of use in the invention can comprise at least 6 µg of neurotrophin.

In another example, supraparticles of use in the invention can comprise between about 2.5 and 10 µg of neurotrophin. In another example, supraparticles can comprise between about 5 and 10 µg of neurotrophin. In another example, supraparticles can comprise between about 6 and 10 µg of neurotrophin. For example, supraparticles can comprise between about 6 and 8 µg of neurotrophin. In another example, supraparticles can comprise between about 5 and 15 µg of neurotrophin. In another example, supraparticles can comprise between about 5 and 20 µg of neurotrophin. In another example, supraparticles can comprise between about 8 and 20 µg of neurotrophin. In another example, supraparticles can comprise between about 8 and 15 µg of neurotrophin.

In an example, supraparticles of use in the invention comprise at least 1.5 µg of payload, wherein the payload has an isoelectric point between 9 and 10. In another example, supraparticles comprise at least 2.0 µg of payload, wherein the payload has an isoelectric point between 9 and 10.

In another example, supraparticles of use in the invention comprise at least 2.5 µg of payload, wherein the payload has an isoelectric point between 9 and 10. In another example, supraparticles comprise at least 3.0 µg of payload, wherein the payload has an isoelectric point between 9 and 10. In another example, supraparticles comprise at least 3.5 µg of payload, wherein the payload has an isoelectric point between 9 and 10. In another example, supraparticles comprise at least 4.0 µg of payload, wherein the payload has an isoelectric point between 9 and 10. In another example, supraparticles comprise at least 5.0 µg of payload, wherein the payload has an isoelectric point between 9 and 10. In another example, supraparticles comprise at least 6.0 µg of payload, wherein the payload has an isoelectric point between 9 and 10. In another example, supraparticles comprise at least 7.0 µg of payload, wherein the payload has an isoelectric point between 9 and 10. In another example, supraparticles comprise at least 8.0 µg of payload, wherein the payload has an isoelectric point between 9 and 10. In another example, supraparticles comprise at least 9.0 µg of payload, wherein the payload has an isoelectric point between 9 and 10. In another example, supraparticles comprise at least 10 µg of payload, wherein the payload has an isoelectric point between 9 and 10. In another example, supraparticles comprise at least 10.5 µg of payload, wherein the payload has an isoelectric point between 9 and 10. In another example, supraparticles comprise at least 11 µg of payload, wherein the payload has an isoelectric point between 9 and 10. In another example, supraparticles comprise at least 11.5 µg of payload, wherein the payload has an isoelectric point between 9 and 10. In another example, supraparticles comprise at least 12 µg of payload, wherein the payload has an isoelectric point between 9 and 10. In another example, supraparticles comprise at least 15 µg of payload, wherein the payload has an isoelectric point between 9 and 10. In another example, supraparticles comprise at least 20 µg of payload, wherein the payload has an isoelectric point between 9 and 10.

For example, supraparticles of use in the invention can comprise at least 6 µg of payload, wherein the payload has an isoelectric point between 9 and 10.

In another example, supraparticles of use in the invention can comprise between about 2.5 and 10 µg of payload, wherein the payload has an isoelectric point between 8 and 10. In another example, supraparticles can comprise between about 5 and 10 µg of payload, wherein the payload has an isoelectric point between 9 and 10. In another example, supraparticles can comprise between about 6 and 10 µg of payload, wherein the payload has an isoelectric point between 9 and 10. For example, supraparticles can comprise between about 6 and 8 µg of payload, wherein the payload has an isoelectric point between 9 and 10. In another example, supraparticles can comprise between about 5 and 20 µg of payload, wherein the payload has an isoelectric point between 9 and 10. In another example, supraparticles can comprise between about 5 and 15 µg of payload, wherein the payload has an isoelectric point between 9 and 10. In another example, supraparticles can comprise between about 8 and 20 µg of payload, wherein the payload has an isoelectric point between 9 and 10. In another example, supraparticles can comprise between about 8 and 15 µg of payload, wherein the payload has an isoelectric point between 9 and 10. For example, the payload can be a neurotrophin which has an isoelectric point between 9 and 10.

In an example, supraparticles of use in the invention having an above referenced payload can be provided in an alginate hydrogel.

Supraparticles of use in the invention loaded with an above exemplified payload can have a pore size selected from the examples discussed below. In an example, supraparticles of use in the invention are microporous. The term "microporous" is used in the context of the present disclosure to refer to particles having a pore size of less than about 2 nm. For example, microporous supraparticles can have a pore size of about 0.5 nm to about 2 nm. In other examples, microporous supraparticles have a pore size of about 1 nm to about 2 nm, about 1.5 nm to about 2 nm. In another example, supraparticles of use in the invention are mesoporous. The term "mesoporous" is used in the context of the present disclosure to refer to particles having pores with diameters between about 2 nm and about 50 nm. For example, mesoporous supraparticles can have a pore size of about 2 nm to about 50 nm. In other examples, mesoporous supraparticles have a pore size of about 2 nm to about 40 nm, about 2 nm to about 30 nm. In another example, supraparticles of use in the invention are macroporous. The term "macroporous" is used in the context of the present disclosure to refer to particles having a pore size of greater than about 50 nm. For example, macroporous supraparticles can have a pore size of about 50 nm to about 500 nm. In other examples, macroporous supraparticles have a pore size of about 50 nm to about 250 nm, about 50 nm to about 150 nm, about 50 nm to about 100 nm.

Supraparticles of use in the invention are comprised of mesoporous nanoparticles. In an example, supraparticles of use in the invention are comprised of nanoparticles having a pore size of about 2 nm to about 50 nm. In other examples, supraparticles are comprised of nanoparticles having a pore size of about 2 nm to about 40 nm, about 2 nm to about 30 nm.

In another example, the mesoporous nanoparticles of the supraparticles of use in the invention have a bimodal pore structure. The term "bimodal" is used in the context of the present disclosure to refer to particles comprising multiple pore sizes, generally a smaller pore size and a larger pore size. In another example, supraparticles of use in the invention are comprised of bimodal nanoparticles having smaller pore sizes of about 2 nm to about 4 nm and larger pore sizes of about 15 nm to about 40 nm. In another example, supraparticles of use in the invention are comprised of bimodal nanoparticles having smaller pore sizes of about 2 nm to about 3 nm and larger pore sizes of about 4 nm to about 40 nm.

In another example, supraparticles of use in the invention are loaded with at least 3 µg of an above referenced payload and are comprised of bimodal nanoparticles having smaller pore sizes of about 2 nm to about 4 nm and larger pore sizes of about 15 nm to about 40 nm. In another example, supraparticles of use in the invention are loaded with at least 5 µg of an above referenced payload and are comprised of bimodal nanoparticles having smaller pore sizes of about 2 nm to about 4 nm and larger pore sizes of about 15 nm to about 40 nm.

In another example, supraparticles of use in the invention are loaded with at least 8 µg of an above referenced payload and are comprised of bimodal nanoparticles having smaller pore sizes of about 2 nm to about 4 nm and larger pore sizes of about 15 nm to about 40 nm. In another example, supraparticles of use in the invention are loaded with at least 10 µg of an above referenced payload and are comprised of bimodal nanoparticles having smaller pore sizes of about 2 nm to about 4 nm and larger pore sizes of about 15 nm to about 40 nm. In these examples, the supraparticles can be provided in an alginate hydrogel.

In an example, supraparticles of use in the invention can have a substantially uniform pore size. In another example, supraparticles of use in the invention comprise variable pore sizes. In this example, pore size may be variable but fall within a particular size range. For example, supraparticles of use in the invention can be predominantly mesoporous. In another example, supraparticles can be predominantly macroporous. In another example, supraparticles of use in the invention are comprised of nanoparticles having a substantially uniform pore size. In another example, supraparticles of use in the invention are comprised of bimodal nanoparticles having substantially uniform small and large pore sizes.

In an example, the supraparticles of use in the invention can have an ordered pore structure. These supraparticles have pores with a regular, three-dimensional spacing. In another example, the supraparticles of use in the invention can have a disordered pore structure. These supraparticles have pores with an irregular, three-dimensional spacing. In another example, supraparticles of use in the invention have a combination of both ordered and disordered pore structures.

It will be appreciated by the person skilled in the art that supraparticle pore size can be measured by, for example, transmission electron microscopy (TEM), scanning electron microscopy (SEM) and X-Ray computed tomography. One of skill in the art can identify supraparticles having the above exemplified pore sizes by measuring the width across the widest point of their three dimensional structure. In an example, the widest point or a pore may be at the surface of the supraparticle.

Supraparticles of the present disclosure may be characterised by the distance between their particles. In an example, the average distance between particles can range from about 80 nm to about 400 nm. In another example, the average distance between colloidal particles ranges from about 90 nm to about 300 nm, about 100 nm to

In an example, supraparticle pores are connected. For example, supraparticles can comprise a series of interconnected pores. In another example, supraparticle pores are not connected. In another example, supraparticles have a combination of both connected and unconnected pores.

Supraparticles of the present disclosure may be characterised by the volume of their pores. In an example, supraparticle pore volume ranges from about 0.5 mLg⁻¹ to about 10 mLg⁻¹. In another example, supraparticles have a pore volume of about 0.8 mLg⁻¹ to about 5 mLg⁻¹, about 1 mLg⁻¹ to about 2.5 mLg⁻¹, about 1.5 mLg⁻¹ to about 2 mLg⁻¹.

Supraparticles of the present disclosure may have a hollow core or a toroidal core. In an example, the internal volume of the supraparticle core is at least about 45%, at least about 55%, at least about 65%, at least about 70%, at least about 75%, at least about 80%, at least about 85%, at least about 90%, at least about 95% of the total volume of the supraparticle. Exemplary methods of producing hollow core supraparticles include acid core processes such as those described in US 4,468,498 and ester core process such as those described in US 5,157,084 and 5,521,253.

Supraparticles of the present disclosure may be characterised by the surface area of their structure. In an example, the surface area of supraparticles range from about 500 m²g⁻¹ to about 1500 m²g⁻¹. In another example, supraparticles have a surface area of between about 5500 m²g⁻¹ and about 1250 m²g⁻¹, about 600 m²g⁻¹ and 1000 m²g⁻¹, about 600 m²g⁻¹ and 700 m²g⁻¹. In an example, the surface area of supraparticles is about 600 m²g⁻¹. In an example, the surface area of supraparticles is about 620 m²g⁻¹.

Supraparticles according to the present disclosure may be characterised by a particular *in-vitro* release profile. In an example, supraparticles release payload for at least 50 days. In an example, supraparticles release payload for at least 100 days. In an example, supraparticles release payload for at least 150 days.

In another example, supraparticles release an initial burst of payload followed by sustained release of payload. For example, supraparticles release a high level of payload for around 3 to 10 days followed by sustained release profile for 30 days or more. In another example, supraparticles release a high level of payload for around 8 to 10 days followed by sustained release profile for 50 days or more.

In an example, the initial burst of payload is 1.2 µg/day for at least 2 days. In another example, the initial burst of payload is 1.3 µg/day for at least 3 days. In another example, the initial burst of payload is 1.4 µg/day for 2 to 4 days. An example of a sustained release profile is at least 0.04 µg/day. Another example of a sustained release profile is at least 0.06 µg/day. Other examples of sustained release profiles are at least 0.07 µg/day and at least 0.1 µg/day.

In another example, supraparticles release at least 2 µg of payload for at least 20 days. In another example, supraparticles release at least 2 µg of payload for at least 30 days. In another example, supraparticles release at least 2 µg of payload for at least 40 days. In another example, supraparticles release at least 4 µg of payload for at least 20 days. In another example, supraparticles release at least 4 µg of payload for at least 30 days. In another example, supraparticles release at least 4 µg of payload for at least 40 days.

In another example, supraparticles release at least 2 µg of neurotrophic factor for at least 20 days. In another example, supraparticles release at least 2 µg of neurotrophic factor for at least 30 days. In another example, supraparticles release at least 2 µg of neurotrophic factor for at least 40 days. In another example, supraparticles release at least 4 µg of neurotrophic factor for at least 20 days. In another example, supraparticles release at least 4 µg of neurotrophic factor for at least 30 days. In another example, supraparticles release at least 4 µg of neurotrophic factor for at least 40 days. For example, supraparticles release at least 2 µg of BDNF for at least 20 days. In other examples, supraparticles can release at least 2 µg of BDNF for at least 40 days. In another example, supraparticles can release at least 4 µg of BDNF for at least 20 days. In another example, supraparticles can release at least 4 µg of BDNF for at least 40 days.

One of skill in the art can measure the *in-vitro* release profile of supraparticles using various methods. Examples of such methods are discussed below in Example 7. For example, supraparticles disclosed herein can be loaded with a labelled payload such as FITC-lysozyme before being incubated in solution such as PBS. Intermittent measurements of fluorescence can be used to determine the level of payload (e.g. µg) released over time.

In an example, supraparticles of use in the invention are produced from nanoparticles having a diameter of between about 1 nm and 100 nm.

Exemplary particles which may form supraparticles include organic particles, inorganic particles, metal particles or a combination thereof. Exemplary organic particles include polymeric particles such as polyglycolic acid (PGA), polylactic acid (PLA), poly(methacryclic acid), poly(ethacrylic acid), polyacrylic acid (PAA), poly(N-isopropylacrylamide), poly(N,N-dimethylacrylamide), polyamides, poly-2-hydroxy butyrate (PHB), gelatines, polycaprolactone (PCL), and poly (lactic-co-glycolic acid) (PLGA). Exemplary inorganic particles include mineral fillers such as heavy fillers or high density fillers, pigments, clays and other synthetic particles. Other exemplary inorganic particles include dense minerals such as barite, hematite, magnesium oxide, inorganic oxides including titanium dioxide, calcium oxide, zinc oxide, magnesium oxide, cerium oxide, zirconium dioxide, and silicon dioxide. In an example, the material is silicon dioxide (i.e. silica). Thus, in such an example, the supraparticles can be referred to as silica supraparticles. Exemplary metal particles include gold, silver, and copper. Supraparticles may comprise the same particles. For example, supraparticles can substantially consist of silica particles. Supraparticles may comprise different particles; for example silica and clay particles. Supraparticles can comprise at least three, at least four, at least 5, at least 6, at least 7, at least 8, at least 9, at least 10 different particles.

Supraparticles may comprise polyelectrolytes or polyelectrolyte material. Examples of such supraparticles are disclosed in WO 2006/037160. In this example, the polyelectrolyte may be a positively charged polyelectrolyte (or have the ability to be positively charged) or a negatively charged polyelectrolyte (or have the ability to be negatively charged) or have a zero net charge.

Supraparticles of use in the invention comprise mesoporous silica nanoparticles.

Supraparticles of the present disclosure can have various shapes. For example, supraparticles can have a spherical shape. Exemplary spherical shapes include spheres and ovoids. In another example, supraparticles have a non-spherical shape. Exemplary non-spherical shapes include dumbbell, hemisphere, disc, tetrahedron, fibre, spherocylinder and irregular shapes. In an example, supraparticles can have an ordered structure. For example, supraparticles may comprise an ordered array.

Spherical supraparticles of the present disclosure may be characterised by their diameter. For example, supraparticles of the present disclosure have a diameter greater than 100 µm. For example, supraparticles of the present disclosure can have a diameter of at least about 150 µm, about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1000 µm. For example, supraparticles can have a diameter of about 550 µm. This diameter of supraparticle is advantageous as it allows for high drug loading while facilitating inner ear delivery via cannula. In other examples, supraparticles can have a diameter of between about 150 µm and about 1000 µm, about 200 µm and about 900 µm, about 300 µm and about 800 µm. In another example, supraparticles can have a diameter of between about 400 µm and about 600 µm. In another example, supraparticles can have a diameter of between about 450 µm and about 550 µm. In another example, supraparticles can have a diameter of between about 520 µm and about 580 µm. In another example, supraparticles can have a diameter of between about 460 µm and about 540 µm. In another example, supraparticles can have a diameter of between about 470 µm and about 530 µm. In another example, supraparticles can have a diameter of between about 480 µm and about 520 µm. In another example, supraparticles can have a diameter of between about 490 µm and about 510 µm. In other examples, supraparticles can be characterised by the width across the widest point of their three dimensional structure. For example, supraparticles can have a width consistent with the above exemplified diameters.

In another example, the supraparticle surface is modified by the addition of functional moieties to enhance the loading of a payload. Any number of functional moieties may be added to the surface of a supraparticle, with the choice of functional moiety being chosen to compliment the payload being loaded. In an example, a moiety such as 3-aminopropyltriethoxysilane (APTS), is grafted onto the surface of silica supraparticle. This introduces an amine functionality that can interact with any carboxyl groups present on a payload. In another example, the supraparticle is modified to bear an overall net charge that enhances loading of the payload (Tan et al. Adv. Mater. (2012) 24, 3362-3366). For example, the surface of the supraparticle can be modified to bear an overall net positive charge, such that loading of a payload bearing an overall net negative charge is enhanced. In another example, the surface of the supraparticle is modified to bear an overall net negative charge, such that loading of a payload bearing an overall net positive charge is enhanced.

The supraparticles of the present disclosure may also comprise cross linked functional moieties. For example, functional moieties may be crosslinked through a chemical reaction. In an example, polyglycolic acid (PGA) molecules are crosslinked by a chemical reaction of the PGA molecules with cystamine (Tan et al. Adv. Mater. (2012) 24, 3362-3366).

### Formulations

Supraparticles of use in the invention may be formulated as a pharmaceutical composition suitable for administration to a subject. Exemplary pharmaceutical compositions may provide supraparticles alone or in combination with a pharmaceutically acceptable carrier, diluent or excipient. In these compositions supraparticles are provided in an amount sufficient to deliver a therapeutically effective amount of payload to a subject. Depending upon the particular route of administration, a variety of acceptable carriers, known in the art may be used, as for example described in Remington's Pharmaceutical Sciences (Mack Publishing Co. N.J. USA, 1991).

Exemplary pharmaceutical compositions may also comprise pharmaceutically acceptable sterile aqueous or nonaqueous solutions, dispersions, suspensions, or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils, and injectable organic esters such as ethyl oleate. Proper fluidity can be maintained, for example, by the use of coating materials such as lecithin, by the maintenance of the required particle size in the case of dispersions, and by the use of surfactants. Such compositions may also contain adjuvants such as preservatives, wetting agents, emulsifying agents, and dispersing agents or antibacterial and antifungal agents.

In another example, supraparticles of use in the invention can be incorporated into slow release or targeted delivery systems. For example, the present inventors have identified that providing supraparticles disclosed herein with an alginate hydrogel slows the release of payload from the supraparticles. Such slow release systems can include foams, gels, drops and sprays for topical administration or a depot for injection or injection cannula. Exemplary slow release systems include polymer matrices, liposomes, and microspheres. Polymer matrices include reservoir based systems where supraparticles are enclosed in porous polymer coatings (Yang and Pierstorff (2012) JALA. 17, 50-58) and monolithic matrix systems where supraparticles are embedded in polymer matrices (Langer R (1990) Science. 249, 1527-1533). Liposomes may be biodegradable and amphiphilic drug delivery systems, which may be formulated using phospholipids and cholesterol. Microspheres may be formulated using biodegradable and biocompatible polymers. In another example, the slow release system can be polysaccharide based. For example, a supraparticle formulation of the invention can comprise an anionic polysaccharide. In an example, the polysaccharide can be alginic acid or a derivative thereof. For example, the supraparticle formulation of the invention can comprise an alginate hydrogel. Various alginic acid derivatives are known in the art. Examples include sodium alginate, potassium alginate and calcium alginate. Other examples include barium alginate and strontium alginate. In an example, the alginic acid is sodium alginate. Accordingly, in an example, the present invention provides a formulation comprising supraparticle of use in the invention as defined herein and sodium alginate. Alginate can be obtained from various sources (e.g. Sigma, FMC Health and Nutrition). In another example, the polysaccharide is an unbranched polysaccharide such as a glycosaminoglycan. For example, the polysaccharide can be hyaluronic acid.

In another example, supraparticles of use in the invention are provided in a formulation that accelerates release of a payload. For example, supraparticles can be provided with Pluronic F127 based (PF 127) hydrogel or an analogue thereof. In an example, supraparticles of use in the invention can be provided with PF 127 based hydrogel.

In another example, supraparticles of use in the invention are provided in a formulation that enhances diffusion across a membrane within the subjects ear such as the tympanic membrane, oval or round windows. For example, such supraparticle formulations may comprise artificial perilymph.

In another example, supraparticles of use in the invention are incorporated or embedded within scaffolds that are subject-compatible and which degrade into products that are not harmful to a subject. These scaffolds house the supraparticles that are to be transplanted into a subject.

A variety of different scaffolds may be used successfully in the practice of the invention. Exemplary scaffolds include, but are not limited to biological, degradable scaffolds. Natural biodegradable scaffolds include collagen, fibronectin, and laminin scaffolds. Suitable scaffolds include polyglycolic acid scaffolds or synthetic polymers such as polyanhydrides, polyorthoesters, and polylactic acid.

### Treatment and administration

The composition of the invention may be provided for use in a method of treating a disease or disorder. Accordingly, in such an example, compositions comprising supraparticles according to the present invention are administered to a subject in an amount effective to treat a disease or disorder in the subject.

The present invention further provides the composition of the invention for use in treating hearing loss. The term "hearing loss" is used in the context of the present disclosure to refer to any reduction in a subject's ability to detect or process sound. Thus, reference to hearing loss encompasses a partial hearing deficit or total inability to hear.

In an example, the hearing loss is characterised as sensorineural hearing loss (SNHL). SNHL is used in the context of the present disclosure to refer to hearing loss resulting from damage to the delicate sensory hair cells within the cochlea, or loss of their synaptic connections with spiral ganglion neurons (SGNs) or dysfunction of the cochlear Schwann cells. In an example, the hearing loss is characterised as presbycusis. In another example, the hearing loss is noise induced. In another example, the hearing loss is disease induced or genetic. In another example, the hearing loss is induced by exposure to ototoxins, for example aminoglycosides.

In an example, the subject is a mammal. In one example, the subject is a human. For example, the human subject can be an adult. In an example, the human subject is a child. Other exemplary mammalian subjects include companion animals such as dogs or cats, or livestock animals such as horses or cows. Terms such as "subject", "patient" or "individual" are terms that can, in context, be used interchangeably in the present disclosure.

In an example, supraparticles of use in the invention are administered intraperitoneally.

The composition of the invention may also be provided for use in methods of delivering a payload to a cell, tissue or organ in a subject via the ear, the method comprising administering said composition to the ear of a subject. In this example, the method may deliver a payload to a cell from a subjects inner ear, middle ear and/or vestibular system. In another example, the method delivers a therapeutic payload to a neural cell, neural tissue or the brain of a subject.

In an example, compositions of the invention are administered onto the tympanic membrane. In this example, compositions may be formulated for topical administration (e.g. drops, gels, foams, sprays).

In another example, compositions of the invention are administered to the "middle ear" cavity. The term "middle ear" in the context of the present disclosure is used to refer to the space between the tympanic membrane and the inner ear. Thus, the middle ear is external to all inner ear tissue. For example, compositions of the invention can be administered to the middle ear via injection through the tympanic membrane. In this example, supraparticles of use in the invention may be administered as a depot injection. In another example, an opening in the tympanic membrane can be produced by a treating clinician to facilitate access of compositions of the invention to the middle ear. When administering compositions of the invention to the middle ear, said compositions can be administered onto the round and/or oval window.

In another example, supraparticles of use in the invention are administered into the inner ear. For example, supraparticles be administered to the cochlea. In an example, supraparticles can be administered to the basal turn of the cochlea. Surgical techniques to gain access to the cochlea or other structures of the inner ear are known in the art. Exemplary techniques for surgically accessing the human cochlea are described in, for example, Clark GM, et al., "Surgery for an improved multiple-channel cochlear implant", Ann Otol Rhinol Laryngol 93:204-7, 1984, and in Clark GM, et al., "Surgical and safety considerations of multichannel cochlear implants in children", Ear and Hearing Suppl. 12:15S-24S, 1991.

Combination of supraparticles of use in the invention with otic intervention is discussed above. In these examples, otic intervention may occur simultaneously with administration of supraparticles. For example, a cochlear device can be implanted simultaneously with supraparticles. However, increased survival of spiral ganglion neurons can improve the utility of a cochlear implant. Thus, it may be desirable to implant a cochlear device after supraparticles have been administered. For example, a cochlear device can be implanted about one month, about two months, about three months, about six months after the supraparticles have been administered. In these examples, additional supraparticles can be administered with the cochlear device.

In an example, a first dose of supraparticles of use in the invention are administered to the subjects cochlea and at least a second dose of supraparticles are administered onto the subjects round and/or oval window(s).

In an example, a therapeutically effective amount of supraparticles of use in the invention is administered to an ear of the subject. In an example, multiple supraparticles are administered to an ear of the subject. For example, at least two, at least three, at least four, at least 5, at least 10, at least 20 supraparticles can be administered to an ear of the subject. In another example, about one to 10 supraparticles are administered. In other examples about two to 9, about three to 8, about four to 7, about 5 to 6 supraparticles are administered to an ear of the subject. In these examples, supraparticles can comprise the same payload. Alternatively, in another example, supraparticles are loaded with different payloads. For example, a supraparticle loaded with a neurotrophic factor and a supraparticle loaded with a steroid may be administered. In another example, a supraparticle loaded with a neurotrophic factor and a supraparticle loaded with an antibiotic may be administered. In another example, a supraparticle loaded with a neurotrophic factor can be administered with an antibiotic.

### Compositions/Kits

Supraparticles of use in the invention can be provided in a kit or pack. For example, a composition of the invention may be packaged in a suitable container with written instructions for treating an inner ear disorder. In an example, a composition of the invention may be provided in a single dose container such as an ear dropper or prefilled syringe.

In one example, the kit comprises a supraparticle of use in the invention for use in methods of treating an inner ear disorder. In another example, the kit comprises a supraparticle of use in the invention for use in methods of treating hearing loss. In an example, the hearing loss is SNHL. In another example, the hearing loss is noise induced hearing loss. In an example, a kit according to the present disclosure further comprises a hearing aid or an implant. Accordingly, in an example, the kit can comprise a supraparticle of use in the invention and a cochlear implant.

### Manufacturing

The present disclosure encompasses a method of manufacturing supraparticles that can be loaded with high levels of payload. In an example, the supraparticles can be manufactured from a composition comprising nanoparticles and alginic acid or a polysaccharide derivative thereof. Various examples of nanoparticles are provided above. In an example, the nanoparticles have a bimodal pore structure. Nanoparticles can be produced using various methods. One such method is described in Cui et al. 2015, ACS Nano, 9, 1571-1580.

In another example, supraparticles according to the present disclosure are produced by electrospraying. Examples of electrospraying are reviewed in Jaworek A., 2007 Powder Technology 1, 18 - 35. An example of electrospraying is also exemplified below. In an example, the present disclosure encompasses a method of manufacturing supraparticles, the method comprising electrospraying a composition comprising nanoparticles and alginic acid or a polysaccharide derivative thereof into a di-cationic aqueous solution.

More specifically, the present invention further provides a method of manufacturing a composition of the invention, the method comprising electrospraying a composition comprising mesoporous silica nanoparticles and alginic acid into a di-cationic aqueous solution and loading the supraparticles so-produced with at least 1.5 µg of a neurotrophic factor.

The present invention further provides a composition of the invention, wherein the composition is obtainable by electrospraying a composition comprising mesoporous silica nanoparticles and alginic acid into a di-cationic aqueous solution and loading the supraparticles so-produced with at least 1.5 µg of a neurotrophic factor.

One of skill in the art will appreciate that electrospraying parameters can be optimized based on the type of the solution used for electrospraying. For example, voltage and flow rates can be optimised to provide supraparticles of a desired size. In an example, the flow rate is about 6-10 mL h⁻¹. In another example, the flow rate is about 7-9 mL h⁻¹. In another example, the flow rate is about 8 mL h⁻¹. In an example, the voltage is between about 10 and 25 KV. In another example, the voltage is between about 11 and 20 KV. In another example, the voltage is between about 12 and 14 KV. In another example, the voltage is about 13 KV.

In an example, supraparticles are produced by electrospraying a nanoparticle solution. In an example, the concentration of nanoparticles in solution is about 20 mg/ml. In an example, the concentration of nanoparticles in solution is about 30 mg/ml. In an example, the concentration of nanoparticles in solution is about 40 mg/ml. In an example, the concentration of nanoparticles in solution is about 50 mg/ml. In an example, the concentration of nanoparticles in solution is about 60 mg/ml.

In another example, supraparticles are produced by electrospraying a nanoparticle solution comprising alginic acid or a derivative thereof. In an example, the nanoparticle solution is prepared from 5 mg mL⁻¹ alginic acid solution. In an example, the nanoparticle solution is prepared from 10 mg mL⁻¹ alginic acid solution. In an example, the nanoparticle solution is prepared from 20 mg mL⁻¹ alginic acid solution. In an example, the nanoparticle solution is prepared from 30 mg mL⁻¹ alginic acid solution. In another example, the nanoparticle solution is prepared from 5 mg mL⁻¹ to 30 mg mL⁻¹ alginic acid solution. In another example, the nanoparticle solution is prepared from 10 mg mL⁻¹ to 30 mg mL⁻¹ alginic acid solution. In another example, the nanoparticle solution is prepared from 20 mg mL⁻¹ to 30 mg mL⁻¹ alginic acid solution.

In an example, the nanoparticle solution is prepared from 5 mg mL⁻¹ alginic acid in water. In an example, the nanoparticle solution is prepared from 10 mg mL⁻¹ alginic acid in water. In an example, the nanoparticle solution is prepared from 20 mg mL⁻¹ alginic acid in water. In an example, the nanoparticle solution is prepared from 30 mg mL⁻¹ alginic acid in water. In another example, the nanoparticle solution is prepared from 5 mg mL⁻¹ to 30 mg mL⁻¹ alginic acid in water. In another example, the nanoparticle solution is prepared from 10 mg mL⁻¹ to 30 mg mL⁻¹ alginic acid in water. In another example, the nanoparticle solution is prepared from 20 mg mL⁻¹ to 30 mg mL⁻¹ alginic acid in water.

In another example, supraparticles are produced by electrospraying a nanoparticle solution comprising alginic acid.

Alginic acid derivatives are not particularly limited so long as they form a gel at a defined temperature. In an example, the alginic acid derivative is a polysaccharide derivative. Alginic acid derivatives include various alginic acid salt forms. Examples include sodium alginate, potassium alginate and calcium alginate. Other examples include barium alginate and strontium alginate. In an example, the alginic acid is sodium alginate. In another example, supraparticles are produced by electrospraying a nanoparticle solution comprising alginic acid.

Alginates of various viscosities may be used to produce supraparticles according to the present disclosure depending on the desired size and shape of supraparticle. For example, alginate having a viscosity of about 20 to 300 mPa*s can be used. In another example, the alginate has a viscosity of about 20 to 200 mPa*s. In an example, the alginate has a viscosity of 20 mPa*s. In another example, the alginate has a viscosity of 100 mPa*s. In another example, the alginate has a viscosity of 200 mPa*s.

In an example, supraparticles are produced by electrospraying a composition comprising nanoparticles into an aqueous solution. In an example, this is a di-cationic aqueous solution. Exemplary di-cationic components include Ca²⁺ and Ba²⁺. For example, the aqueous solution can comprise calcium chloride. In another example, the aqueous solution comprises barium chloride.

In an example, supraparticles are produced by electrospraying a composition wherein the concentration of alginate in the composition is 5 mg mL⁻¹ to 30 mg mL⁻¹, the concentration of nanoparticles in the composition is 20 mg mL⁻¹ to 50 mg mL⁻¹ and the voltage is 10 kV to 25 kV. In another example, supraparticles are produced by electrospraying a composition wherein the concentration of alginate in the composition is 10 mg mL⁻¹ to 30 mg mL⁻¹, the concentration of nanoparticles in the composition is 30 mg mL⁻¹ to 50 mg mL⁻¹ and the voltage is 11 kV to 21 kV. In another example, supraparticles of use in the invention are produced by electrospraying a composition wherein the concentration of alginate in the composition is 20 mg mL⁻¹ to 30 mg mL⁻¹, the concentration of nanoparticles in the composition is 35 mg mL⁻¹ to 45 mg mL⁻¹ and the voltage is 12 kV to 14 kV. In these examples, the flow rate can be 8 mL h⁻¹.

In another example, supraparticles of use in the invention are produced by electrospraying a composition wherein the concentration of alginate in the composition is 30 mg mL⁻¹, the concentration of nanoparticles in the composition is 40 mg mL⁻¹, the voltage is 13kV, and the flow rate is 8 mL h⁻¹.

In an example, supraparticles manufactured using methods defined herein are subjected to calcination to remove alginic acid or a derivative thereof. In an example, calcination is performed at around 500°C. In another example, calcination is performed at around 550 °C. In another example, calcination is performed at around 600°C. . In another example, calcination is performed at around 650 °C.. In another example, calcination is performed at around 700°C. In an example, calcination is performed for about 6 to about 30 hours. In another example, calcination is performed for about 10 hours. In another example, calcination is performed for about 20 hours. In another example, calcination is performed for about 30 hours.

### EXAMPLES

### EXAMPLE 1 - Nanoparticle production

Mesoporous silica nanoparticles (MS-NP) ware produced using methods based on those described in Cui et al. 2015, ACS Nano, 9, 1571-1580. 1.1g Cetyltrimethylammonium bromide (CTAB) was completely dissolved in 50ml Milli-Q with stirring. 4.3g of Poly(acrylic acid) solution (PAA, M_{w}=250kDa, 35wt% solution in water) was subsequently added with vigorous stirring for 20 mins at room temperature (25°C) until a clear solution was obtained. 3.5ml ammonium hydroxide solution (28-30%) was then added to the solution with vigorous stirring, resulting in a milky suspension. 4.46 ml of Tetraethyl orthosilicate (TEOS) was then added after stirring for 20 min. The solution was stirred for a further 15 min before transferring the mixture into a Teflon-sealed autoclave, which was left at 90°C for 48h.

The as-synthesized MS-NP were washed with ethanol once, water twice and ethanol twice and finally dried at 90°C. The organic templates were removed by calcination at 550°C for 30 h.

### EXAMPLE 2 - Starting Supraparticle manufacturing process - Process A

Mesoporous silica (MS) nanoparticles, were prepared according to Wang et al. (2010) Chem Mater. 22, 3829-3831. MS nanoparticles were dispersed in Milli-Q water with a particle concentration of 5 wt% and briefly sonicated to form a stable colloidal suspension. A 0.5 to 2.0 µL aliquot of the MS nanoparticle dispersion was then applied to a flat surface, which was pre-covered with a paraffin film. The droplets were dried under air flow to drive assembly of the MS nanoparticles into mesoporous silica supraparticles (capillary force MS-SPs) via capillary force action. The size of the capillary force MS-SPs was controlled by the volume of the nanoparticle dispersion applied in the droplet.

Under capillary force, the MS colloids self-assembled into a compact structure to form MS-SPs. The capillary force MS-SPs were then removed from the paraffin film and transferred into a ceramic container, and annealed at 923 K to enhance mechanical stability of the capillary force MS-SPs. Capillary force MS-SPs were then loaded with payload. About 1.33 µg protein was loaded per particle. The capillary force MS-SPs were shown to have a bimodal pore structure (2-3 nm and 15-30 nm) and the macropores within the capillary force MS-SP, the space between the densely packed nanoparticles, was 100-200 nm.

### EXAMPLE 3 - Modified manufacturing process - Process B

80 mg of MS-NPs powder was added into 2 ml of alginic acid sodium salt solution (30 mg mL⁻¹ in water). The resulting solution was sonicated for 1 hour until the MS-NPs distributed uniformly in alginic acid sodium salt solution.

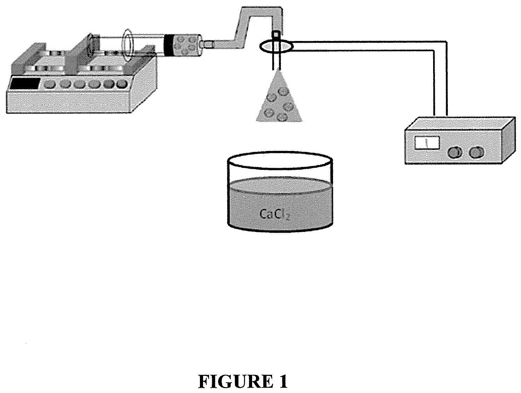

To form large pore MS-SPs (^{L}MS-SPs), the sonicated solution was added into a 3 mL plastic syringe and positioned in a syringe pump, with liquid being electrosprayed into a bath of calcium chloride solution (1 wt% prepared in water) using flow rates around 8 mL h⁻¹ (electrospray setup shown in Figure 1). Droplet size was controlled by applying an electric field between the end of the tubing and the calcium chloride solution. The alginate beads MS-SPs (^{L}MS-SPs^{alg}) were collected from the calcium chloride bath and loaded with payload. Significantly enhanced drug-loading loading performance was observed for these particles (^{L}MS-SPs) compared to those produced by process A: about 7.8 µg protein per particle, with improved mechanical stability.

### EXAMPLE 4 - Modified manufacturing process - Process C

MS-SPs (^{L}MS-SP^{alg}) were produced using the method described in Example 3. Alginic acid sodium salt was removed by calcination at 650°C for 30 h. This step removed all organic components and left behind only the mesoporous silica nanoparticles (MS-SP) and trace amounts of calcium and sodium chloride. MS-SPs were then loaded with payload. Significantly enhanced drug-loading performance was observed for these particles compared to those produced by process A: about 7.8 µg protein per particle.

Supraparticles were also made from nanoparticles without pores (non porous ^{N}MS-SPs^{alg}; ^{N}MS-SPs^{alg}) and nanoparticles having pore sizes <2 nm (small pore MS-^{s}SPs^{alg}; ^{s}MS-SPs^{alg}). When alginate was removed from these supraparticles drug-loading performance was significantly reduced (Table 1).

Maximal drug loading of MS-SP produced by process B and process C is summarized in Table 1.

**Table 1: Loading of MS-SP produced by process B and process C**

| Particle type | qₘₐₓ (ug/particle) |
|---|---|
| non porous ^{N}MS-SPs^{alg} | qₘₐₓ=8.215 |
| non porous ^{N}MS-SPs | qₘₐₓ=2.009 |
| small pore ^{S}MS-SPs^{alg} | qₘₐₓ= 10.06 |
| small pore ^{S}MS-SPs | qₘₐₓ=2.802 |
| large pores ^{L}MS-SPs^{alg} | qₘₐₓ=9.245 |
| large pores ^{L}MS-SPs | qₘₐₓ=7.843 |

### EXAMPLE 5 - Supraparticle release properties

### Lysozyme

FITC-lysozyme-loaded SPs were prepared for *in vitro* release studies by incubating sterilized SPs with 100 µL of FITC-lysozyme solution (0.2 mg mL⁻¹ in Milli-Q water). Lysozyme is a good model protein to mimic the neurotrophin BDNF (as lysozyme is cheap and readily available while BDNF is expensive) because it shares similar physicochemical properties (lysozyme, M_{w} = 14.3 ± 0.5 KDa, R_{H} = 18.9 ± 0.25 Å and pI = 11; BDNF, M_{w} = 13 KDa, R_{H} = 24.0 ± 3.2 Å and pI = 10).

*In vitro* release profile of lysozyme loaded MS-SPs are shown in Figure 4 parts a) and b) and Figure 7 part a). Release profiles of MS-SP produced by process A and C are similar. Although MS-SP produced by process C are loaded with significantly higher levels of labelled lysozyme. Payload release is significantly reduced for MS-SP produced by process B compared to MS-SP produced by processes A and C.

### Zeta potential

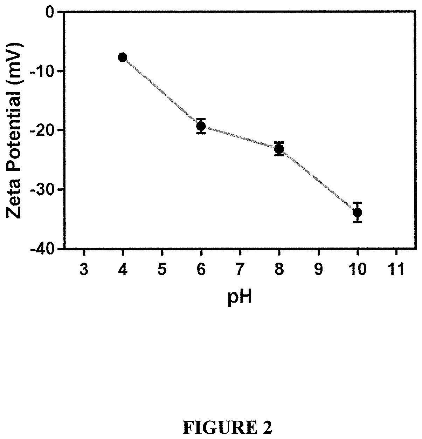

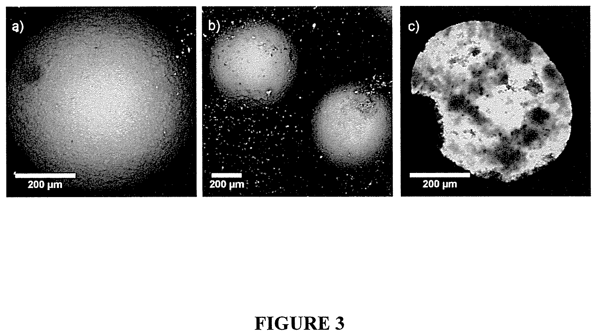

MS-SPs were then loaded with fluorescein-labelled lysozyme (FITC-lysozyme). As shown in Figure 2, MS-SPs exhibited negative zeta potentials ranging from ~-7.6 mV to ~-33.9 mV as the pH value increased from 4 to 10. Positively charged lysozyme and BDNF can therefore be loaded into MS-SPs with the help of electrostatic driving forces. Confocal microscopy images (Figure 3a, b) showed FITC-lysozyme loaded onto the surface of MS-SPs. However, as the size of the MS-SPs is quite large (hundreds of micrometers in diameter) standard laser scanning confocal microscopy is not suitable for imaging the internal structure of the SPs. However, upon fracturing the MS-SPs with a scalpel, the inside could be imaged and it was found that FITC-lysozyme was also observed in the porous internal structures of the MS-SPs (Figure 3c).

### Additional assessment of loading capacity

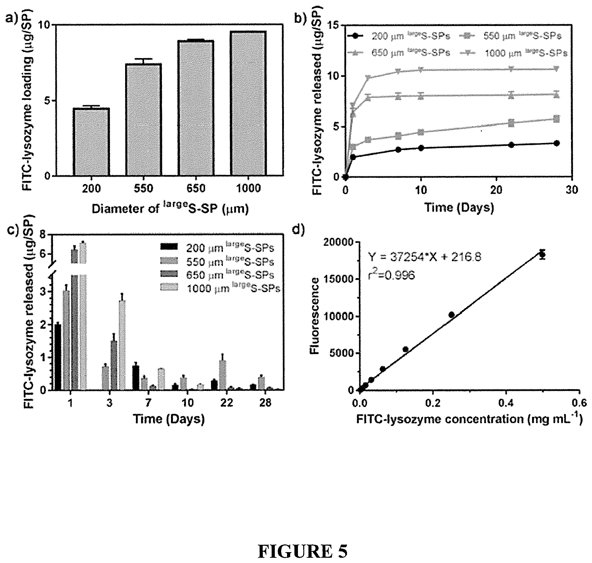

The loading capacity for different types of SPs was then investigated using FITC-lysozyme at different loading concentrations with 3 days incubation time (Figure 4). In general, as the concentration of FITC-lysozyme increased, the amount of drug loading increased. The results show that the alginate-containing ^{N}MS-SPs^{alg}, ^{S}MS-SPs^{alg} and ^{L}MS-SPs^{alg} had higher loading capacities than the alginate-removed ^{N}MS-SPs, ^{S}MS-SPs and ^{L}MS-SPs (at comparable concentrations). This may be due to increased electrostatic interactions between positively charged FITC-lysozyme and negatively charged alginate around neutral pH value. Additionally, at low FITC-lysozyme loading concentration (< 0.4 mg mL⁻¹), ^{L}MS-SPs^{alg} had a similar drug loading capacity as non porous MS-SPs^{alg} and ^{S}MS-SPs^{alg}, but when the concentration was higher (> 0.4 mg mL⁻¹), more FITC-lysozyme could be loaded into ^{L}MS-SPs^{alg} compared to ^{N}MS-SPs^{alg} and ^{S}MS-SPs^{alg}. For alginate-removed MS-SPs a similar trend was observed: ^{L}MS-SPs could load more FITC-lysozyme than ^{N}MS-SPs and ^{S}MS-SPs. These results show that large porous structures (in ^{L}MS-SPs and ^{L}MS-SPs^{alg}) are key factors to improve drug loading, likely by providing additional surfaces and hence loading and capacity when outer particle surfaces and intra-particle areas (in the SP structures) have been fully saturated by the drug. Additionally, the loading capacity also depends on the diameter of MS-SPs where the bigger MS-SPs (1000 µm) had larger loading capacity than the smaller MS-SPs (200 µm) (Figure 5).

The experimentally determined maximum loading amount of FITC-lysozyme into ^{N}MS-SPs and ^{S}MS-SPs^{alg} were about 3 µg per SP and 2 µg per SP respectively, which were significantly lower than ^{L}MS-SPs with approximately 15 µg per SP (FITC-lysozyme concentration was 1.5 mg mL⁻¹, and loading time was 3 days). Besides, the experimentally determined maximum loading amount of FITC-lysozyme into ^{N}MS-SPs and ^{S}MS-SPs were about 2 µg per SP and 1 µg per SP respectively, which were also dramatically less than ^{L}MS-SPs with approximately 10 µg per SP (FITC-lysozyme concentration was 5.0 mg mL⁻¹, and loading time was 3 days).

The drug loading efficiency of six different SPs is shown in Figure 4c and 4d. As the loading concentration of FITC-lysozyme increased, more FITC-lysozyme could be loaded into SPs.

### BDNF

MS-SPs were sterilized by soaking them in 100 µl of ethanol (80 vol/vol%) for 4 hours prior to rinsing with 100 µl of Milli-Q water six times. MS-SPs were then washed once in 0.1 M phosphate buffered saline (PBS). MS-SPs were loaded by placing them in an Eppendorf tube containing 15 µl of BDNF (Geneway, BDNF Human Protein, Cat. # 10-663-45078) solution (1 mg/ml of BDNF) and incubating them at ambient room temperature for three days with occasional hand shaking. Remarkably, loading of about 10 µg BDNF was achieved.

### EXAMPLE 6 - Supraparticle cytotoxicity

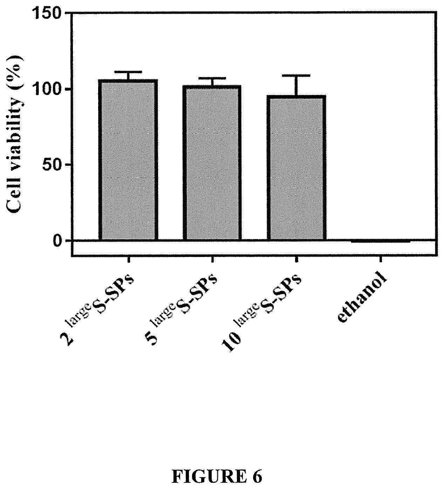

To investigate if MS-SPs could be applied as biocompatible drug carriers, *in vitro* cytotoxicity studies of MS-SPs were performed. Human brain glioblastoma cells (U87MG cell line) were incubated with MS-SPs and cell viability was assessed by an Alamar Blue assay. As shown in Figure 6, MS-SPs were non-toxic even when the number of MS-SPs was increased to ten per well (1×10⁴ cells per well). Typical *in vivo* treatment scenarios of similar particle systems suggest the administration of 1-8 SPs per inner ear.

### EXAMPLE 7 - Drug release

Based on the results of loading capacity, release studies for FITC-lysozyme (loading time 3 days, loading concentration 0.2 mg mL⁻¹) were performed by incubating ten ^{L}MS-SPs and ten MS-SPs^{alg} in PBS (pH 7.4) at 37 °C.

FITC-lysozyme-loaded SPs for *in vitro* release studies were prepared by sterilizing the SPs, followed by incubation of either ten MS-SPs or ten MS-SPs^{alg} with 100 µL of 'low concentration' FITC-lysozyme solution (0.2 mg mL⁻¹ in Milli-Q water). After three days, the supernatant was removed and 100 µL of phosphate buffered saline (PBS, pH 7.4) was added into each tube and incubated at 37 °C. At defined time intervals (over 150 days), 90 µL of solution was collected and replaced with fresh PBS. The fluorescence of the collected samples was measured with an Infinite M200 microplate reader and using a standard curve, the concentration of FITC-lysozyme in the supernatant could then be determined.

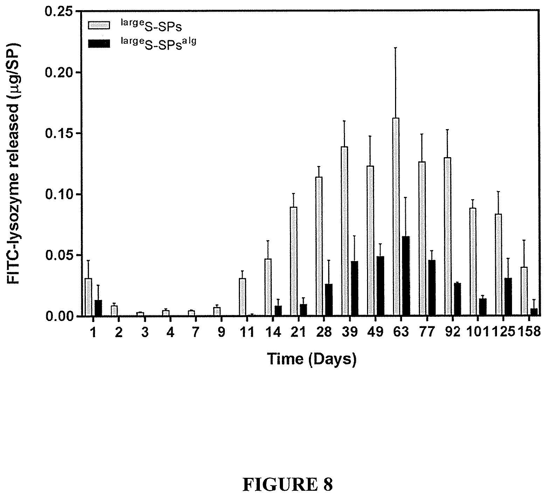

The FITC-lysozyme loading following loading in the low concentration solution were 1.89 ± 0.02 µg and 1.93 ± 0.01 µg per SP for ^{L}MS-SPs and ^{L}MS-SPs^{alg}, respectively. As shown in Figure 7a, >1 µg of FITC-lysozyme per MS-SP was released over more than 150 days, while a lower amount of FITC-lysozyme (~0.4 µg per SP) was released from MS-SPs^{alg} over this time. A possible reason for the lower amount of FITC-lysozyme released from the latter is the presence of alginate, which potentially can block the large pores of the silica primary particles or strongly interact with the lysozyme, preventing or inhibiting lysozyme release. The individual values of measured FITC-lysozyme release at each time point (which was used to prepare the cumulative results presented in Figure 7) is shown in Figure 8.

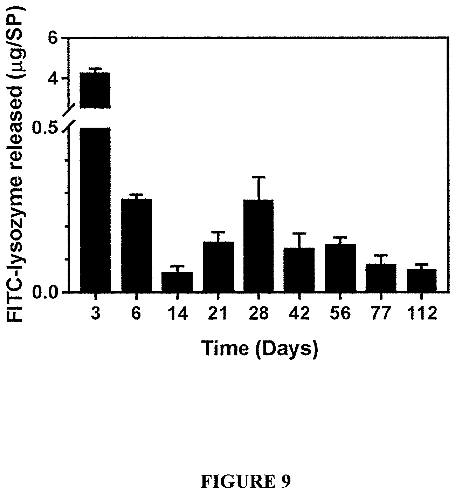

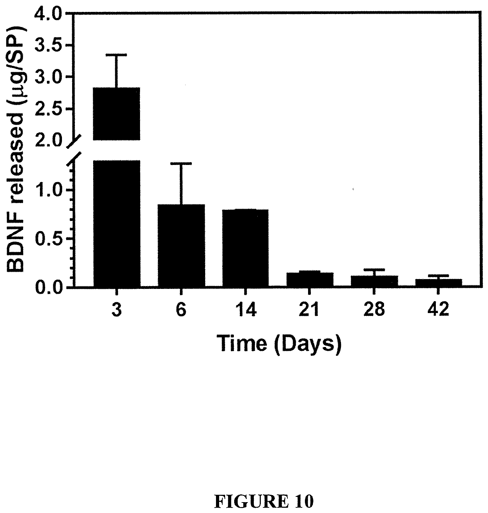

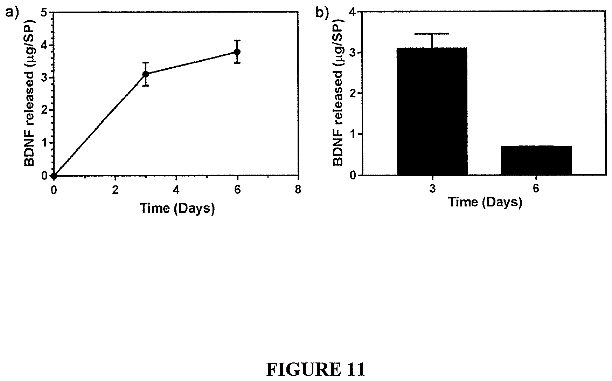

As the loading concentration of drug used for loading the SP increased, more drug could be loaded into ^{L}MS-SPs. Therefore, drug release profiles for both FITC-lysozyme and BDNF at a higher incubation concentration (1.0 mg mL⁻¹) were investigated. The FITC-lysozyme loading amount at this concentration (1.0 mg mL⁻¹) was 6.49 ± 0.48 µg per SP. The release profile for FITC-lysozyme was observed in two release stages: burst release in the first three days and sustained release over more than 110 days (Figure 7b). Around 4.2 µg FITC-lysozyme per SP was released in the burst stage. After 3 days, ~2.3 µg FITC-lysozyme (loaded amount - amount released during burst release) remained loaded in the SPs and a more sustained release behavior was subsequently observed. The individual values of FITC-lysozyme released at each time point was tens to hundreds of nanograms per SP (Figure 9). As shown in Figure 7c, the *in vitro* BDNF release profile was similar to that for FITC-lysozyme, with a burst release observed in the first three days followed by a more sustained release for over 40 days. The cumulative released value of the SPs after 40 days was -4.68 µg of drug, and the weight of each SP was ~0.05 mg. Thus, the observed cumulative release was about 93.6 µg mg⁻¹. This is a 4000-fold improvement compared to what has recently been observed for nanoporous silica nanoparticles. The individual values of BDNF released at each time point ranged from tens to hundreds of nanograms per SP (Figure 10). The amount of BDNF released was also measured by a MicroBCA assay (Figure 11), with similar results.

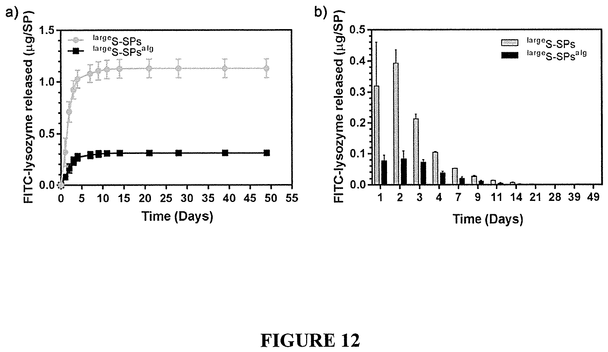

In order to investigate potential methods to further tune drug release behavior and aid in surgical delivery in the inner ear, *in vitro* drug release studies of MS-SPs and MS-SPs^{alg} incorporated within PF127 hydrogels were tested. PF127 hydrogels form as viscous liquids at cold temperatures (e.g. 4 °C) and gel at body temperature (37 °C). In these studies, most of the FITC-lysozyme was observed to be released from both MS-SPs and MS-SPs^{alg} (both in PF127 hydrogels) by approximately day 14 (Figure 12) indicating accelerated release kinetics. Therefore, the supraparticle-PF127 system is suitable when shorter term drug release is desired (-1 week), while SPs without the PF127 hydrogel is more suitable for longer term (several months) drug release.

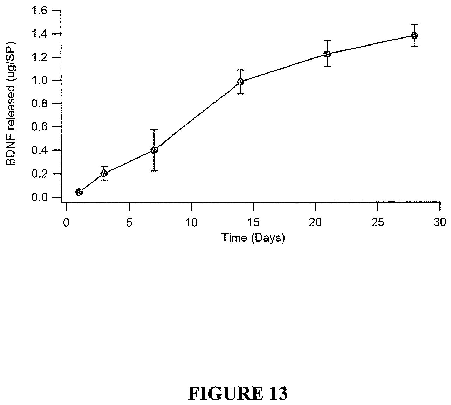

*In vitro* experiments were also carried out to establish BDNF release kinetics over time when the SPs were loaded approximately 50%. Consistent with the data from the lysozymes the BDNF release was more linear over the first 4 weeks (e.g there was no burst release; Figure 13).

### EXAMPLE 8 - Degradation of Supraparticles

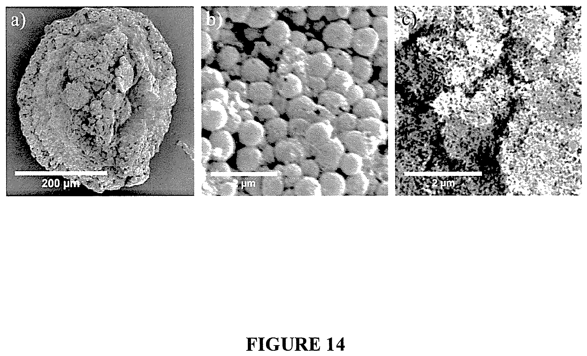

Porous silica particles that can be degraded in biological environments (e.g. into silicic acid, which can be excreted through urine) and are of interest for diverse biomedical applications. To investigate degradation rates, MS-SPs were incubated for 150 days (PBS, pH 7.4, 37 °C) (Figure 14). A -55% decrease in diameter of the MS-SPs was observed, and the shape/morphology of the MS-SPs also changed substantially. The silica primary particles on the surface of the MS-SPs changed in both morphology and size.

### EXAMPLE 9 - Pharmacokinetics

I*n vivo* studies were carried out to determine the drug payload and clearance of neurotrophin delivered in a supraparticle implanted into the ear after 4hr, 3day and 7days of implantation. The objective was to determine the amount of neurotrophin remaining in the cochlea over time.

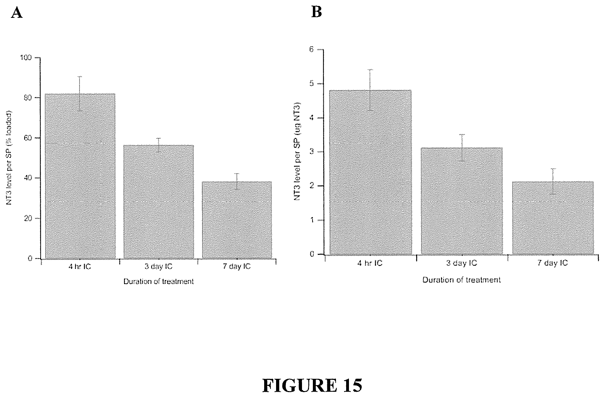

1 SP containing radiolabelled neurotrophin-3 was implanted into each cochlea. Cochleae were harvested following 4hrs (n=5), 3d (n=7) or 7d (n=4). Whole cochlear gamma counts were measured to determine the clearance of neurotrophin-3 over time. The remaining amount of neurotrophin-3 (% of loaded) and the total in µg is shown in Figure 15. After 1 week of implantation each SP contained ~2ug of neurotrophin-3 (~40%) of initial loaded amount.

Further experiments (n=2) were carried out to examine clearance of neurotrophin-3 following round window delivery. 3 days after implantation - whole cochlear measurements were again used to determine the clearance of neurotrophin-3 from the round window membrane. A similar level of clearance was observed using the round window compared to the intracochlear delivery site (47.4% of neurotrophin-3 remaining following round window delivery compared to 56% for intracochlear delivery).

These data show that extended release of neurotrophin-3 can be achieved with high levels of neurotrophin-3 still available even after 1 week of treatment.

### REFERENCES

Clark GM, et al. (1984) Ann Otol Rhinol Laryngol 93:204-207
Clark GM, et al. (1991) Ear and Hearing Suppl. 12:15S-24S
Cui et al. (2015) ACS Nano 9:1571-1580
Jaworek (2007) Powder Technology 176(1), 18-35
Langer R (1990) Science. 249, 1527-1533
Remington's Pharmaceutical Sciences (Mack Publishing Co. N.J. USA, 1991)
Tan et al. (2012) Adv. Mater. 24: 3362-3366
Wang et al. (2009) J. Mater. Chem. 19: 6451-6464
Wang et al. (2010) Chem Mater. 22: 3829-3831
Yang and Pierstorff (2012) JALA. 17, 50-58

## Claims

1. A composition comprising a supraparticle, said supraparticle comprising mesoporous silica nanoparticles and at least one payload, wherein said payload is at least 1.5 µg of a neurotrophic factor.

2. The composition of claim 1, wherein the supraparticle comprises at least 5 µg of payload.

3. The composition of claim 1, wherein the supraparticle comprises 1.5 µg to 10 µg of payload.

4. The composition according to any one of claims 1 to 3, wherein the supraparticle comprises pores having a diameter of at least 50 nm, preferably at least 60 nm.

5. The composition according to any one of claims 1 to 4, wherein the supraparticles have a disordered pore structure.

6. The composition according to any one of claims 1 to 5, wherein the supraparticle is comprised of nanoparticles having a bimodal pore structure, preferably wherein the nanoparticles bimodal pore structure has a large pore diameter greater than 30 nm.

7. The composition according to any one of claims 1 to 6, wherein the neurotrophic factor
(i) has an isoelectric point between 8 and 10;
(ii) is BDNF; or
(ii) is neurotrophin-3.

8. The composition according to any one of claims 1 to 7, wherein the supraparticle comprises at least 2, at least 3, at least 4, or at least 5 different payloads.

9. The composition according to any one of claims 1 to 8, wherein the composition is provided as a slow release formulation, preferably comprising a glycogen hydrogel.

10. The composition according to any one of claims 1 to 9, wherein the supraparticles are manufactured by electrospraying a composition comprising said nanoparticles and alginic acid into a di-cationic aqueous solution.

11. The composition according to any one of claims 1 to 10, comprising alginic acid [(C₆H₈O₆)ₙ], preferably alginic acid sodium salt [Na(C₆H₈O₆)n].

12. The composition according to any one of claims 1 to 11, for use in treating a disorder, wherein the disorder is hearing loss, and preferably wherein said hearing loss is characterised as sensorineural hearing loss (SNHL), presbycusis or noise induced.

13. The composition of any one of claims 1 to 11, for use in promoting survival of spiral ganglion neurons in an ear of a subject.

14. The composition of any one of claims 1 to 13, wherein the supraparticle has a diameter of 150 µm to 1000 µm.

15. The composition of any one of claims 1 to 14, wherein the composition is obtainable by electrospraying a composition comprising said nanoparticles and alginic acid into a di-cationic aqueous solution and loading the supraparticles so-produced with at least 1.5 µg of a neurotrophic factor.

16. A method of manufacturing the composition according to any one of claims 1 to 15, comprising electrospraying a composition comprising said nanoparticles and alginic acid into a di-cationic aqueous solution and loading the supraparticles so-produced with at least 1.5 µg of a neurotrophic factor.

17. The method of claim 16, further comprising subjecting the supraparticles to calcination to remove alginic acid, preferably wherein calcination is performed at around 650 °C or for about 6 to about 30 hours.

## Patentansprüche

1. Zusammensetzung, die ein Suprapartikel umfasst, wobei das Suprapartikel mesoporöse Silica-Nanopartikel und mindestens eine Ladekapazität umfasst, wobei die Ladekapazität mindestens 1,5 µg eines neurotrophen Faktors beträgt.

2. Zusammensetzung nach Anspruch 1, wobei das Suprapartikel mindestens 5 µg Ladekapazität umfasst.

3. Zusammensetzung nach Anspruch 1, wobei das Suprapartikel 1,5 µg bis 10 µg Ladekapazität umfasst.

4. Zusammensetzung nach einem der Ansprüche 1 bis 3, wobei das Suprapartikel Poren mit einem Durchmesser von mindestens 50 nm, vorzugsweise mindestens 60 nm, umfasst.

5. Zusammensetzung nach einem der Ansprüche 1 bis 4, wobei die Suprapartikel eine ungeordnete Porenstruktur aufweisen.

6. Zusammensetzung nach einem der Ansprüche 1 bis 5, wobei das Suprapartikel aus Nanopartikeln mit einer bimodalen Porenstruktur besteht, wobei die bimodale Porenstruktur der Nanopartikel vorzugsweise einen großen Porendurchmesser von mehr als 30 nm aufweist.

7. Zusammensetzung nach einem der Ansprüche 1 bis 6, wobei der neurotrophe Faktor
(i) einen isoelektrischen Punkt zwischen 8 und 10 aufweist;
(ii) BDNF ist; oder
(iii) Neurotrophin-3 ist.

8. Zusammensetzung nach einem der Ansprüche 1 bis 7, wobei das Suprapartikel mindestens 2, mindestens 3, mindestens 4 oder mindestens 5 verschiedene Ladekapazitäten umfasst.

9. Zusammensetzung nach einem der Ansprüche 1 bis 8, wobei die Zusammensetzung als langsam freisetzende Formulierung bereitgestellt wird, die vorzugsweise ein Glykogen-Hydrogel umfasst.

10. Zusammensetzung nach einem der Ansprüche 1 bis 9, wobei die Suprapartikel durch Elektrosprühen einer Zusammensetzung, die die Nanopartikel und Alginsäure umfasst, in eine dikationische wässrige Lösung hergestellt werden.

11. Zusammensetzung nach einem der Ansprüche 1 bis 10, umfassend Alginsäure [(C₆H₈O₆)n], vorzugsweise Alginsäure-Natriumsalz [Na(C₆H₈O₆)n].

12. Zusammensetzung nach einem der Ansprüche 1 bis 11 zur Verwendung bei der Behandlung einer Erkrankung, wobei es sich bei der Erkrankung um einen Hörverlust handelt, und wobei der Hörverlust vorzugsweise als sensorineuraler Hörverlust (SNHL), Altersschwerhörigkeit oder lärminduzierter Hörverlust gekennzeichnet ist.

13. Zusammensetzung nach einem der Ansprüche 1 bis 11 zur Verwendung bei der Förderung des Überlebens von Spiralganglionneuronen in einem Ohr eines Subjekts.

14. Zusammensetzung nach einem der Ansprüche 1 bis 13, wobei das Suprapartikel einen Durchmesser von 150 µm bis 1000 µm aufweist.

15. Zusammensetzung nach einem der Ansprüche 1 bis 14, wobei die Zusammensetzung durch Elektrosprühen einer Zusammensetzung, die die Nanopartikel und Alginsäure umfasst, in eine dikationische wässrige Lösung und Beladen der so hergestellten Suprapartikel mit mindestens 1,5 µg eines neurotrophen Faktors erhältlich ist.

16. Verfahren zur Herstellung der Zusammensetzung nach einem der Ansprüche 1 bis 15, umfassend Elektrosprühen einer Zusammensetzung, die die Nanopartikel und Alginsäure umfasst, in eine dikationische wässrige Lösung und Beladen der so hergestellten Suprapartikel mit mindestens 1,5 µg eines neurotrophen Faktors.

17. Verfahren nach Anspruch 16, das weiter Unterziehen der Suprapartikel einer Kalzinierung umfasst, um Alginsäure zu entfernen, wobei Kalzinierung vorzugsweise bei etwa 650°C oder für etwa 6 bis etwa 30 Stunden durchgeführt wird.

## Revendications

1. Composition comprenant une supraparticule, ladite supraparticule comprenant des nanoparticules de silice mésoporeuse et au moins une charge utile, dans laquelle ladite charge utile est d'au moins 1,5 µg d'un facteur neurotrophique.

2. Composition selon la revendication 1, dans laquelle la supraparticule comprend au moins 5 µg de charge utile.

3. Composition selon la revendication 1, dans laquelle la supraparticule comprend 1,5 µg à 10 µg de charge utile.

4. Composition selon l'une quelconque des revendications 1 à 3, dans laquelle la supraparticule comprend des pores présentant un diamètre d'au moins 50 nm, de préférence d'au moins 60 nm.

5. Composition selon l'une quelconque des revendications 1 à 4, dans laquelle les supraparticules présentent une structure de pores désordonnée.

6. Composition selon l'une quelconque des revendications 1 à 5, dans laquelle la supraparticule est composée de nanoparticules présentant une structure de pores bimodale, de préférence dans laquelle la structure de pores bimodale des nanoparticules présente un grand diamètre de pores supérieur à 30 nm.

7. Composition selon l'une quelconque des revendications 1 à 6, dans laquelle le facteur neurotrophique
(i) présente un point isoélectrique compris entre 8 et 10 ;
(ii) est le BDNF ; ou
(iii) est la neurotrophine-3.

8. Composition selon l'une quelconque des revendications 1 à 7, dans laquelle la supraparticule comprend au moins 2, au moins 3, au moins 4 ou au moins 5 charges utiles différentes.

9. Composition selon l'une quelconque des revendications 1 à 8, dans laquelle la composition est fournie sous forme de formulation à libération lente, comprenant de préférence un hydrogel de glycogène.

10. Composition selon l'une quelconque des revendications 1 à 9, dans laquelle les supraparticules sont fabriquées par électronébulisation d'une composition comprenant lesdites nanoparticules et de l'acide alginique en une solution aqueuse di-cationique.

11. Composition selon l'une quelconque des revendications 1 à 10, comprenant de l'acide alginique [(C₆H₈O₆)ₙ], de préférence un sel de sodium d'acide alginique [Na(C₆H₈O₆)ₙ].

12. Composition selon l'une quelconque des revendications 1 à 11, destinée à être utilisée dans le traitement d'un trouble, dans laquelle le trouble est une perte auditive, et de préférence dans laquelle ladite perte auditive est **caractérisée** comme étant une surdité de perception (SNHL), une presbyacousie ou induite par le bruit.

13. Composition selon l'une quelconque des revendications 1 à 11, destinée à être utilisée pour favoriser la survie des neurones du ganglion spiral dans une oreille d'un sujet.

14. Composition selon l'une quelconque des revendications 1 à 13, dans laquelle la supraparticule présente un diamètre de 150 µm à 1 000 µm.

15. Composition selon l'une quelconque des revendications 1 à 14, dans laquelle la composition peut être obtenue par électronébulisation d'une composition comprenant lesdites nanoparticules et de l'acide alginique en une solution aqueuse di-cationique et chargement des supraparticules ainsi produites avec au moins 1,5 µg d'un facteur neurotrophique.

16. Procédé de fabrication de la composition selon l'une quelconque des revendications 1 à 15, comprenant l'électronébulisation d'une composition comprenant lesdites nanoparticules et de l'acide alginique en une solution aqueuse di-cationique et le chargement des supraparticules ainsi produites avec au moins 1,5 µg d'un facteur neurotrophique.

17. Procédé selon la revendication 16, comprenant en outre la soumission des supraparticules à une calcination pour éliminer l'acide alginique, de préférence dans lequel la calcination est effectuée à environ 650°C ou pendant environ 6 à environ 30 heures.
